# EUROPEAN PATENT APPLICATION

(11) **EP 4 389 142 A2**
(43) Date of publication of application: **26.06.2024**
(21) Application number: 24160069.1
(22) Date of filing: 18.06.2018
(51) Int. Cl.: A61K 39/00

(54) **USE OF ANTI CD70 ANTIBODY ARGX-110 TO TREAT ACUTE MYELOID LEUKAEMIA**

(30) Priority: 16.06.2017 GB 201709677
(62) Divisional of application: 18732064.3
(71) Applicant: argenx BV, 9052 Zwijnaarde (Ghent) (BE); University of Bern, 3012 Bern (CH)
(72) Inventor: LEUPIN, Nicolas, B-9052 Gent (BE); VAN ROMPAEY, Luc, B-9052 Gent (BE); DE HAARD, Hans, B-9052 Gent (BE); OCHSENBEIN, Adrian, B-9052 Gent (BE); RIETHER, Carsten, B-9052 Gent (BE)
(74) Representative: Boult Wade Tennant LLP

(57) **Abstract**

Methods of treating acute myeloid leukaemia (AML) or myelodysplastic syndrome (MDS) are provided, as are compositions and combinations suitable for use in said methods.

## Description

### FIELD OF INVENTION

The present invention relates to methods of treating acute myeloid leukaemia (AML) and myelodysplastic syndrome (MDS), and compositions and combinations suitable for use in said methods.

### BACKGROUND

Acute myeloid leukaemia (AML) is a heterogeneous disease characterized by uncontrolled clonal expansion of hematopoietic progenitor cells. AML is the most common acute leukaemia affecting adults, with a yearly incidence in European adults of 5 to 8 cases per 100,000 individuals, and a steep increase in the population aged over 70 years where the incidence reaches 15-25/100,000 per year.

The survival statistics published by Cancer Research UK for AML diagnosed in England between 2008 and 2010, for all ages, show that approximately 20% of subjects survive for 5 years or more after diagnosis. Prognostic factors for a poor outcome include the subject's age, treatment-induced AML, and history of myelodysplastic syndromes or another antecedent haematological disorder. The 5-year survival rate in those aged 65 or older, is approximately 5%.

Chemotherapy, either single agent or combination treatments, is used to treat most types of leukaemia. Under appropriate conditions, high-dose chemotherapy followed by hematopoietic stem cell transplantation may also be used. Around 60% to 70% of adults with AML will attain complete remission (CR) status following appropriate induction therapy, and about 45% of those who attain CR can be expected to survive 3 or more years and may be cured (American Cancer Society). However, the side effects of chemotherapy can place significant burden on the patient, and many patients are not fit for standard intensive chemotherapy. Alternative therapies for AML are therefore desirable.

### SUMMARY OF INVENTION

CD70 is a cell surface antigen normally expressed in a small subset of activated B- and T-lymphocytes as well as on mature dendritic cells, and involved in lymphocyte differentiation and survival signalling upon binding to its cognate cell surface receptor, CD27. CD70-induced signalling of CD27 results in increased production and activation of regulatory T cells, which express CD27.

CD70 expression is low or absent from normal tissues, including all vital organs. CD70 is over-expressed in several tumour types, often together with CD27 in haematological malignancies suggesting its involvement in proliferation and survival of the malignant cells. CD70 also appears to play a role in evasion of immune surveillance by inducing Tregs, thus promoting tumour growth.

Inhibition of the CD70-CD27 signalling pathway on regulatory T cells is believed to impede regulatory T cells recruitment and/or activation thereby potentially restoring a subject's immuno-surveillance in the tumour microenvironment.

The present invention demonstrates for the first time the effective treatment of AML and MDS in humans with an anti-CD70 antibody. Treatment according to the invention is effective even after a single dose of anti-CD70 antibody, and even at a surprisingly low dose. Not only is anti-CD70 antibody administered as a monotherapy surprisingly effective, but it is demonstrated herein that a combination therapy of an anti-CD70 antibody together with a nucleoside metabolic inhibitor (NMI) results in further efficacy. As used herein, a combination therapy according to the invention does not require simultaneous administration of the two or more active agents, or for the two or more agents to be formulated into a single composition.

AML patients treated with the therapies provided herein exhibited greater than 90% response rates, with 2 in every 3 patients achieving complete remission. These results represent a significant advance in AML therapy for all patients.

As demonstrated in the accompanying Examples, treatment according to the invention significantly reduces the percentage of bone marrow blasts in a patient. As already noted, treatment according to the invention significantly reduced the number of blasts to the point that patients entered complete remission. Also, significantly reducing the percentage of bone marrow blasts in a patient is important if the patient is to receive a successful haematopoietic stem cell transplant (HSCT), a treatment that can be curative. Importantly, treatment according to the invention has resulted in a patient progressing to transplant.

Notably, treatment according to the invention effectively treats AML without any observed increased toxicity compared to that reported for conventional NMI treatment.

This is significant for all AML patients. Standard intensive chemotherapy for AML comes with significant toxicity, with many patients experiencing severe side effects. The limited toxicity associated with the effective treatment according to the present invention demonstrates the potential for the therapies described herein to provide an improved AML treatment for all patients.

The surprising efficacy of treatment according to the invention is of further importance given the nature of the patients recruited to the trial. The treated patients were not eligible for standard chemotherapy because they were not fit enough to tolerate the toxicity associated with conventional intensive chemotherapy.

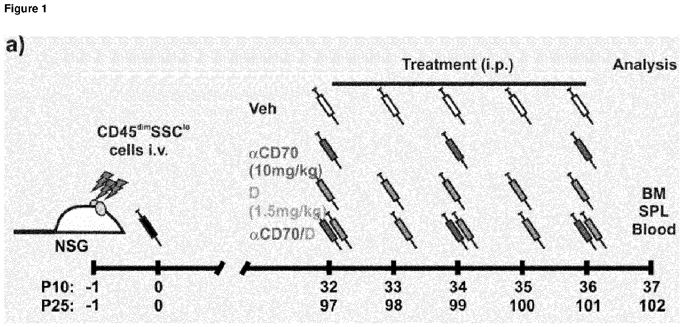

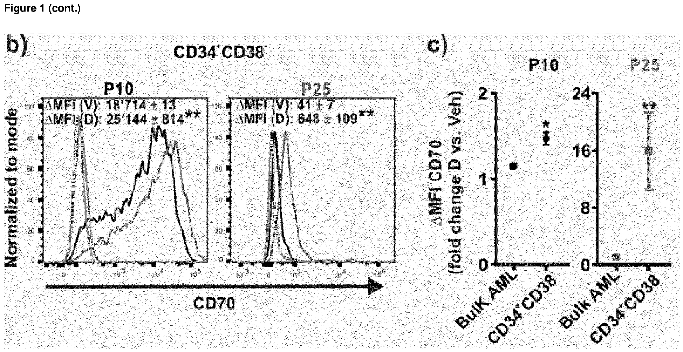

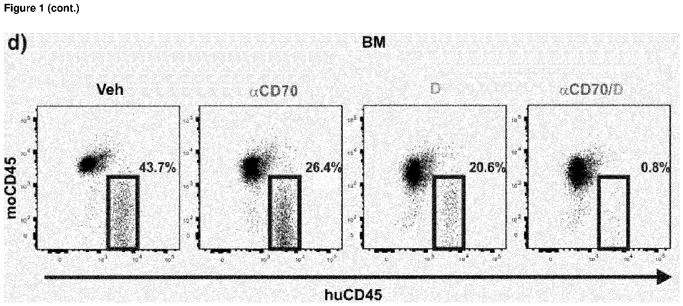

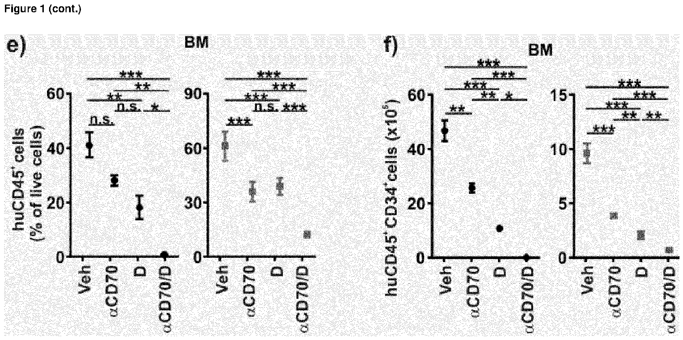

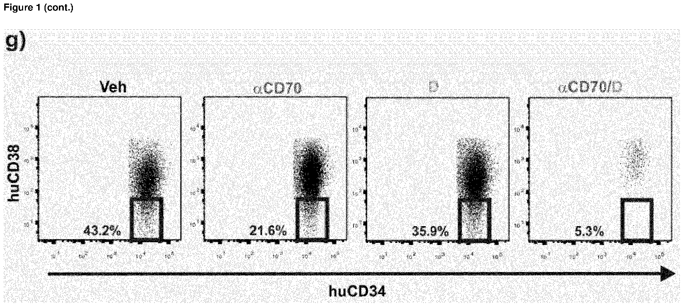

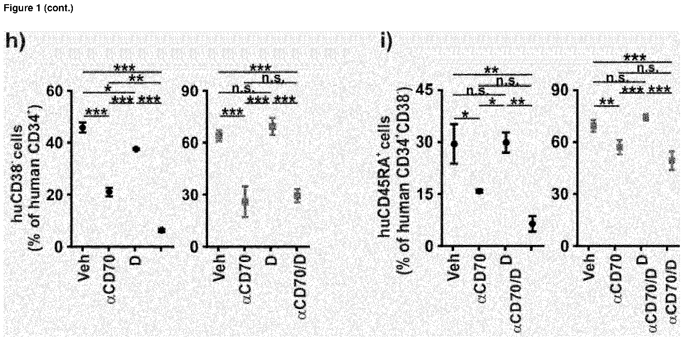

Since standard intensive chemotherapy is thought to be required to sufficiently reduce bone marrow blasts, HSCT is not currently available to these patients. However, as demonstrated herein, monotherapy and combination therapy according to the present invention significantly reduces AML blasts in patients who cannot receive standard intensive chemotherapy. Accordingly, the therapies provided in accordance with the invention open up the prospect of a successful HSCT in a population of patients for whom HSCT was not previously recommended. A further significant advantage of the fact that anti-CD70 antibodies can effectively treat AML both as a monotherapy and also in a potent combination therapy with a nucleoside metabolic inhibitor is that it allows a range of treatment models to be used. For example, the combination therapy with a nucleoside metabolic inhibitor (NMI) provides a potent therapy arising from the effects of the CD70 antibodies being augmented by the upregulation of CD70 on blasts in response to the NMI (*in vitro* and *in vivo* (Figure 1)). This provides an effective therapy with reduced toxicity compared to standard intensive chemotherapy, particularly important for patients who would not be healthy enough to tolerate standard intensive chemotherapy. Monotherapy with CD70 antibodies provides an effective treatment without the need even for a NMI. This further reduces the toxicity by avoiding the effect of the NMI on non-blast cells, which can lead to a reduced risk of the patient developing cytopenias. By reducing the risk of cytopenias, the patient is less at risk from infection and requires fewer blood transfusions.

A further treatment model made available by the present invention is an "induction" and "maintenance" model. That is, an induction treatment using the combination of anti-CD70 plus nucleoside metabolic inhibitor (with an optional loading dose of anti-CD70) can be used to potently decrease AML blast percentages in the bone marrow. The patient can then transition to a maintenance treatment of anti-CD70 alone or in combination with lower doses of NMI. This model has the advantage that the decrease in blasts can be maintained without the need to expose the patient to potential accumulated toxicity from prolonged administration of the nucleoside metabolic inhibitor.

Moreover, such maintenance treatment with anti-CD70 antibody alone specifically suppresses blast cells (which express CD70) with minimal effect on other cell types (which have minimal or no CD70 expression). Since nucleoside metabolic inhibitor doses can be stopped or reduced, cytotoxic pressure on non-blast cell types is reduced, allowing these cells to proliferate. This has the advantage of reducing the risk of cytopenias arising from prolonged NMI treatment, as other cell types (e.g. platelets, erythrocytes, neutrophils) can recover whilst AML blasts are targeted by the CD70 antibody.

Furthermore, it is demonstrated herein that administration of an anti-CD70 antibody, alone or in combination with a NMI, promotes LSC differentiation into myeloid cells. Promotion of such differentiation reduces the population of LSCs able to self-renew. LSCs contribute significantly to the propagation and maintenance of disease, providing a self-renewing pool of malignant cells. Thus, by inducing differentiation of LSCs, administration of an anti-CD70 antibody, alone or in combination with a NMI, decreases the LSC population thereby increasing the prospect of remission and reducing the risk of relapse.

Accordingly, in one aspect the invention provides a method for treating acute myeloid leukaemia (AML) or myelodysplastic syndrome (MDS) in a subject comprising: administering to the subject one or more doses of an anti-CD70 antibody or antigen-binding fragment thereof.

In a further aspect is provided a method for reducing the percentage of blasts in the bone marrow and/or peripheral blood of a subject with AML or MDS, the method comprising administering to the subject one or more doses of an anti-CD70 antibody or antigen-binding fragment thereof.

In a further aspect is provided a method for preparing a subject with AML or MDS for haematopoietic stem cell transplant (HSCT) comprising administering to the subject one or more therapeutically effective doses of an anti-CD70 antibody or antigen-binding fragment thereof.

In a further aspect the invention provides an anti-CD70 antibody or antigen binding fragment thereof for use in a method for treating acute myeloid leukaemia (AML) or myelodysplastic syndrome (MDS) in a subject comprising administering to the subject one or more doses of the anti-CD70 antibody or antigen-binding fragment thereof.

In a further aspect the invention provides an anti-CD70 antibody or antigen binding fragment thereof for use in a method for reducing AML blast cells in a subject, the method comprising administering to the subject one or more doses of the anti-CD70 antibody or antigen-binding fragment thereof.

In a further aspect the invention provides an anti-CD70 antibody for use in a method for preparing a subject with AML or MDS for haematopoietic stem cell transplant (HSCT) comprising administering to the subject one or more doses of an anti-CD70 antibody or antigen-binding fragment thereof.

In a further aspect the invention provides a pharmaceutical composition for use in a method for treating acute myeloid leukaemia (AML) or myelodysplastic syndrome (MDS) in a subject comprising administering to the subject one or more doses of an anti-CD70 antibody or antigen-binding fragment thereof, the pharmaceutical composition comprising the anti-CD70 antibody or antigen-binding fragment thereof and a pharmaceutically acceptable excipient or carrier.

In a further aspect is provided a pharmaceutical composition for use in a method for reducing AML blast cells in a subject, the method comprising administering to the subject one or more doses of an anti-CD70 antibody or antigen-binding fragment thereof.

In a further aspect is provided a pharmaceutical composition for use in a method for preparing a subject with AML or MDS for haematopoietic stem cell transplant (HSCT) comprising administering to the subject one or more doses of an anti-CD70 antibody or antigen-binding fragment thereof.

In a further aspect is provided an anti-CD70 antibody or antigen binding fragment thereof for use in treating AML, wherein the antibody or antigen binding fragment is administered in combination with a hypomethylating agent, preferably azacitdine.

In certain embodiments of the methods of the invention the anti-CD70 antibody or antigen-binding fragment thereof is administered at a dose in the range from 0.1 mg/kg to 25 mg/kg per dose, optionally at a dose in the range from 1 mg/kg to 20 mg/kg. In certain embodiments the anti-CD70 antibody or antigen-binding fragment thereof is administered at a dose of 1 mg/kg, 3 mg/kg, 10 mg/kg or 20 mg/kg. In certain preferred embodiments the anti-CD70 antibody or antigen-binding fragment thereof is administered at a dose of 10 mg/kg.

In certain embodiments of all aspects of the methods of the invention the method further comprises the administration of a nucleoside metabolic inhibitor (NMI), for example a hypomethylating agent (HMA). In certain embodiments the NM! is azacitidine or decitabine.

In certain embodiments the methods according to the invention comprise: (i) a first stage comprising the administration of an anti-CD70 antibody and nucleoside metabolic inhibitor as a combination therapy in accordance with the dosage regimens described herein, and (ii) a second stage comprising the administration of an anti-CD70 antibody in accordance with the dosage regimens described herein and the administration of a lower dose of the nucleoside metabolic inhibitor than the dose of nucleoside metabolic inhibitor administered in the first stage.

In certain embodiments, the subject is not eligible for standard intensive chemotherapy prior to treatment according to the invention.

In certain embodiments the methods further comprise performing a haematopoietic stem cell transplant on the subject.

In certain embodiments the patient is 60 years or older, optionally 75 years old or older.

In certain embodiments the invention further comprises the administration of one or more active agents selected from an anti-CD33 antibody, an anti-CD123 antibody, an E-selectin inhibitor, a FLT3 inhibitor, a cyclin-dependent kinase inhibitor, a BCL-2 inhibitor, an aminopeptidase inhibitor and a JAK/STAT inhibitor as part of a combination therapy.

In a further aspect the invention provides a combination comprising an anti-CD70 antibody or antigen binding fragment thereof and a NMI. In certain embodiments, the NMI is a hypomethylating agent. In certain preferred embodiments, the hypomethylating agent is azacitidine or decitabine, preferably azacitidine.

In a further aspect the invention provides a combination comprising an anti-CD70 antibody or antigen binding fragment thereof and a NMI for use in a method of treating AML or MDS. In certain embodiments, the NMI is a hypomethylating agent. In certain preferred embodiments, the hypomethylating agent is azacitidine or decitabine, preferably azacitidine.

In a further aspect the invention provides a combination comprising an anti-CD70 antibody or antigen binding fragment thereof and a NMI for use in a method according to the invention.

In all aspects of the invention, the anti-CD70 antibody may inhibit the interaction of CD70 with its receptor CD27. In certain embodiments, the anti-CD70 antibody or antigen-binding fragment thereof may inhibit CD70-CD27 binding. In certain embodiments, the anti-CD70 antibody or antigen-binding fragment thereof may inhibit CD70-CD27 induced signalling.

In all aspects of the invention the anti-CD70 antibody may possess antibody effector function, for example antibody dependent cell-mediated cytotoxicity (ADCC), complement dependent cytotoxicity (CDC) and/or antibody dependent cellular phagocytosis (ADCP). In certain embodiments, the anti-CD70 antibody or antigen-binding fragment thereof may deplete CD70-expressing cells, for example via antibody effector function.

In certain embodiments, the anti-CD70 antibody may be a modified antibody, for example an antibody drug conjugate (ADC). As described elsewhere herein, ADCs are antibodies conjugated to an active agent such as a cytotoxic agent. ADCs may also possess one or more antibody effector functions in addition to delivering the active agent to a target.

In certain embodiments of all aspects of the invention, the anti-CD70 antibody comprises a variable heavy chain domain (VH) and a variable light chain domain (VL), wherein the VH and VL domains comprise the CDRs:
HCDR3 comprising or consisting of SEQ ID NO:3 (DAGYSNHVPIFDS)
HCDR2 comprising or consisting of SEQ ID NO:2 (DINNEGGTTYYADSVKG)
HCDR1 comprising or consisting of SEQ ID NO:1 (VYYMN)
LCDR3 comprising or consisting of SEQ ID NO:7 (ALFISNPSVE)
LCDR2 comprising or consisting of SEQ ID NO:6 (NTNTRHS), and
LCDR1 comprising or consisting of SEQ ID NO:5 (GLKSGSVTSDNFPT).

In certain embodiments the anti-CD70 antibody or antigen-binding fragment comprises a VH domain at least 80%, at least 90%, at least 95%, at least 98%, at least 99% identical to SEQ ID No 4 and/or comprises a VL domain at least 80% at least 90%, at least 95%, at least 98%, at least 99% identical to SEQ ID No 8. For embodiments wherein the domains of the antibodies or antigen binding fragments are defined by a particular percentage sequence identity to a reference sequence, the VH and/or VL domains may retain identical CDR sequences to those present in the reference sequence such that the variation is present only within the framework regions.

In certain embodiments the anti-CD70 antibody is an IgG1 antibody.

In all aspects of the invention, the anti-CD70 antibody is preferably ARGX-110.

In all aspects of the invention, a preferred embodiment is a combination of ARGX-110 and azacitidine.

In all aspects of the invention, the subject or patient is a human subject or patient.

### The invention can also be understood with reference to the following numbered clauses:

1. A method for treating acute myeloid leukaemia (AML) or myelodysplastic syndrome (MDS) in a subject comprising:
   administering to the subject one or more doses of an anti-CD70 antibody or antigen-binding fragment thereof.
2. A method for reducing the percentage of blasts in the bone marrow and/or peripheral blood of a subject with AML or MDS, the method comprising administering to the subject one or more therapeutically effective doses of an anti-CD70 antibody or antigen-binding fragment thereof.
3. A method for preparing a subject with AML or MDS for haematopoietic stem cell transplant (HSCT) comprising administering to the subject one or more doses of an anti-CD70 antibody or antigen-binding fragment thereof.
4. A method according to any preceding clause, wherein the method reduces the percentage of blasts in the bone marrow of the subject.
5. The method according to any preceding clause, wherein the method reduces the percentage of blasts in the peripheral blood of the subject.
6. The method of any preceding clause wherein the anti-CD70 antibody or antigen-binding fragment thereof is administered at a dose in the range from 0.1 mg/kg to 25 mg/kg per dose.
7. The method of any preceding clause wherein the anti-CD70 antibody or antigen-binding fragment thereof is administered at a dose in the range from 1 mg/kg to 20 mg/kg per dose.
8. The method of any preceding clause wherein the anti-CD70 antibody or antigen-binding fragment thereof is administered at a dose of 10 mg/kg.
9. The method according to any preceding clause, wherein each dose of the anti-CD70 antibody or antigen-binding fragment thereof is separated by 10-20 days, optionally 12-18 days, optionally 14-17 days.
10. The method of any preceding clause further comprising administering a nucleoside metabolic inhibitor to the subject.
11. The method of clause 10 wherein the nucleoside metabolic inhibitor is a hypomethylating agent, optionally azacitidine or decitabine.
12. The method of clause 10 or 11 wherein the nucleoside metabolic inhibitor is azacitidine.
13. The method of any one of clauses 10-12, wherein the nucleoside metabolic inhibitor is administered at a dose in the range of 50-100mg/m² per day
14. The method of any one of clauses 10-13, wherein the nucleoside metabolic inhibitor is administered in a dosing period of a daily dose for 5-9 days.
15. The method of clause 14, wherein the nucleoside metabolic inhibitor is administered according to a dosage regimen of repeated dosing periods, wherein the end of one dosing period and the start of the next dosing period are separated by 18-25 days.
16. The method of any one of clauses 10-15, wherein the first dose of nucleoside metabolic inhibitor is administered 7-21 days after the first dose of anti-CD70 antibody or antigen binding fragment thereof.
17. The method of any one of clauses 10-16, wherein one of the daily doses of the nucleoside metabolic inhibitor is administered on the same day as a dose of the anti-CD70 antibody or antigen-binding fragment thereof.
18. The method according to any one of clauses 10-17, wherein the method comprises:
   i) a first stage comprising the administration of an anti-CD70 antibody and nucleoside metabolic inhibitor according to any of clauses 10-17, and
   ii) a second stage comprising the administration of an anti-CD70 antibody according to any one of clauses 1-9 and the administration of a lower dose of the nucleoside metabolic inhibitor than the dose of nucleoside metabolic inhibitor administered in the first stage.
19. The method of any preceding clause wherein the subject is not eligible for standard intensive chemotherapy prior to treatment.
20. The method of any preceding clause further comprising the step of conducting a hematopoietic stem cell transplant on the subject.
21. The method of any preceding clause wherein the subject is 60 years old or older, optionally 75 years old or older.
22. The method according to any preceding clause further comprising administering to the subject one or more active agents selected from an anti-CD33 antibody, an anti-CD123 antibody, an E-selectin inhibitor, a FLT3 inhibitor, a cyclin-dependent kinase inhibitor, a BCL-2 inhibitor, an aminopeptidase inhibitor and a JAK/STAT inhibitor.
23. The method according to any preceding clause further comprising monitoring the subject's blast count.
24. The method according to any preceding clause which induces partial response (PR), complete response with incomplete haematological recovery (CRi), or complete response (CR).
25. The method according to any preceding clause which induces a minimal residual disease status that is negative.
26. The method according to any preceding clause which increases survival versus standard of care agents for which the subject would be eligible.
27. A method according to any preceding clause wherein the anti-CD70 antibody or antigen-binding fragment thereof inhibits CD70-CD27 binding.
28. A method according to any preceding clause wherein the anti-CD70 antibody or antigen-binding fragment thereof depletes CD70-expressing cells.
29. A method according to any preceding clause, wherein the anti-CD70 antibody comprises a variable heavy chain domain (VH) and a variable light chain domain (VL), wherein the VH and VL domains comprise the CDRs:
   HCDR3 comprising or consisting of SEQ ID NO:3 (DAGYSNHVPIFDS)
   HCDR2 comprising or consisting of SEQ ID NO:2 (DINNEGGTTYYADSVKG)
   HCDR1 comprising or consisting of SEQ ID NO:1 (VYYMN)
   LCDR3 comprising or consisting of SEQ ID NO:7 (ALFISNPSVE)
   LCDR2 comprising or consisting of SEQ ID NO:6 (NTNTRHS), and
   LCDR1 comprising or consisting of SEQ ID NO:5 (GLKSGSVTSDNFPT).
30. A method according to any preceding clause wherein the anti-CD70 antibody or antigen-binding fragment comprises a VH domain at least 80% identical to SEQ ID NO: 4 and/or comprises a VL domain at least 80% identical to SEQ ID NO: 8.
31. A method according to any preceding clause wherein the anti-CD70 antibody is an IgG1 antibody.
32. A method according to any preceding clause wherein the anti-CD70 antibody is ARGX-110.
33. An anti-CD70 antibody for use in a method according to any preceding clause.
34. A pharmaceutical composition for use in a method according to any preceding clause, wherein the pharmaceutical composition comprises an anti-CD70 antibody and a pharmaceutically acceptable excipient or carrier.
35. An anti-CD70 antibody or antigen binding fragment thereof for use in treating AML, wherein the antibody or antigen binding fragment is administered in combination with a hypomethylating agent, preferably azacitdine.
36. An antibody or pharmaceutical composition for use according to any one of clauses 33-35 wherein the anti-CD70 antibody or antigen-binding fragment thereof inhibits CD70-CD27 binding.
37. An antibody or pharmaceutical composition for use according to any one of clauses 33-36 wherein the anti-CD70 antibody or antigen-binding fragment thereof depletes CD70-expressing cells.
38. An antibody or pharmaceutical composition for use according to any one of clauses 33-37 wherein the anti-CD70 antibody or antigen-binding fragment comprises a variable heavy chain domain (VH) and a variable light chain domain (VL), wherein the VH and VL domains comprise the CDRs:
   HCDR3 comprising or consisting of SEQ ID NO:3 (DAGYSNHVPIFDS)
   HCDR2 comprising or consisting of SEQ ID NO:2 (DINNEGGTTYYADSVKG)
   HCDR1 comprising or consisting of SEQ ID NO:1 (VYYMN)
   LCDR3 comprising or consisting of SEQ ID NO:7 (ALFISNPSVE)
   LCDR2 comprising or consisting of SEQ ID NO:6 (NTNTRHS), and
   LCDR1 comprising or consisting of SEQ ID NO:5 (GLKSGSVTSDNFPT).
39. An antibody or pharmaceutical composition for use according to any one of clauses 33-38 wherein the anti-CD70 antibody or antigen-binding fragment comprises a VH domain at least 80% identical to SEQ ID No 4 and/or comprises a VL domain at least 80% identical to SEQ ID No 8.
40. An antibody or pharmaceutical composition for use according to any one of clauses 33-39 wherein the anti-CD70 antibody is an IgG1 antibody.
41. An antibody or pharmaceutical composition for use according to any one of clauses 33-40 wherein the anti-CD70 antibody is ARGX-110.
42. A combination comprising an anti-CD70 antibody or antigen binding fragment thereof and a NMI.
43. A combination according to clause 42, wherein the NMI is a hypomethylating agent.
44. A combination according to clause 43, wherein the hypomethylating agent is azacitidine.
45. A combination according to any one of clauses 42-44, wherein the anti-CD70 antibody or antigen-binding fragment thereof inhibits CD70-CD27 binding.
46. A combination according to any one of clauses 42-45 wherein the anti-CD70 antibody or antigen-binding fragment thereof depletes CD70-expressing cells.
47. A combination according to any one of clauses 42-46, wherein the anti-CD70 antibody comprises a variable heavy chain domain (VH) and a variable light chain domain (VL), wherein the VH and VL domains comprise the CDRs:
   HCDR3 comprising or consisting of SEQ ID NO:3 (DAGYSNHVPIFDS)
   HCDR2 comprising or consisting of SEQ ID NO:2 (DINNEGGTTYYADSVKG)
   HCDR1 comprising or consisting of SEQ ID NO:1 (VYYMN)
   LCDR3 comprising or consisting of SEQ ID NO:7 (ALFISNPSVE)
   LCDR2 comprising or consisting of SEQ ID NO:6 (NTNTRHS), and
   LCDR1 comprising or consisting of SEQ ID NO:5 (GLKSGSVTSDNFPT).
48. A combination according to any one of clauses 42-47 wherein the anti-CD70 antibody or antigen-binding fragment comprises a VH domain at least 80% identical to SEQ ID No 4 and/or comprises a VL domain at least 80% identical to SEQ ID No 8.
49. A combination according to any one of clauses 42-48 wherein the anti-CD70 antibody is an IgG1 antibody.
50. A combination according to any one of clauses 42-49 wherein the anti-CD70 antibody is ARGX-110.
51. A combination according to any one of clauses 42-50 for use in a method for treating AML or MDS.
52. A combination according to any one of clauses 42-50 for use in a method according to any of clauses 1-32.

### BRIEF DESCRIPTION OF FIGURES

- Figure 1: aCD70/decitabine combination therapy eradicates human CD34+CD38- AML stem/progenitor cells in murine xenografts. (a-i) 5×10⁶ FACS-purified CD45^{dim}SSC^{lo} cells from BM of newly diagnosed AML patients (patient P10 and P21) were injected intravenously into the tail vein of sub-lethally irradiated NSG mice. After engraftment (day 32 (P10) and day 97 (P25) after transplantation), mice were randomized and subjected to treatment with control mAb and 10mg/kg aCD70 mAb (41D12-D) intraperitoneally (total of 3 injections) or decitabine and (1.5mg/kg/day) for five consecutive days alone or in combination. One day after the last treatment, animals were sacrificed and blood, spleen and BM were analyzed. (a) Experimental setup. (b) CD70 expression on CD34⁺CD38⁻ AML stem/progenitor cells. Isotype is depicted in grey; CD70 staining in black (P10) and blue (P25). Solid lines represent vehicle and dashed lines, decitabine treatment on AML stem/progenitor cells. ΔMFI: MFI staining - MFI isotype (c) Fold change CD70 expression on non-CD34⁺CD38⁻ bulk and CD34⁺CD38⁻AML stem/progenitor cells (Veh vs. D). (d) Representative FACS plots of engraftment of human CD45⁺ AML cells in the BM of PDX mice. (e) Frequency of human CD45⁺ AML cells in BM of PDX AML mice. (f) Absolute numbers of CD45^{dim}SSC^{lo}lin⁻CD90⁻CD34⁺AML stem/progenitor cells in the BM. (g) Representative FACS plots and (h) quantification indicating the frequency of CD38⁻ AML cells within CD45^{dim}SSC^{lo}lin⁻CD90⁻CD34⁺AML stem/progenitor cell population. (i) Frequency of CD45RA⁺ cells in the CD45^{dim}SSC^{lo}lin-CD90-CD34⁺CD38⁻AML stem/progenitor cell population. Data are represented as mean ±S.D. Statistics: (b, c) Student's t-test; (e, f, h, i) One-way-ANOVA; Tukey's post-test; P<0.05; **, *P*<0.01 ; ***, P<0.001.

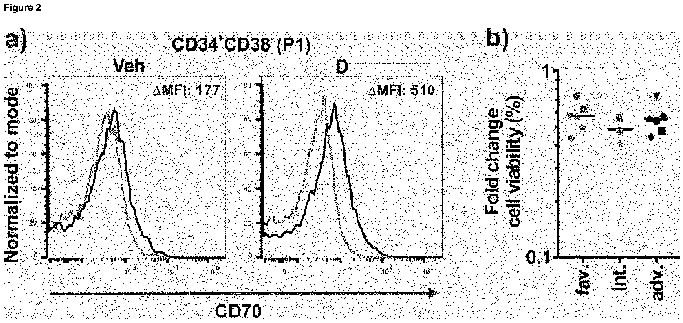

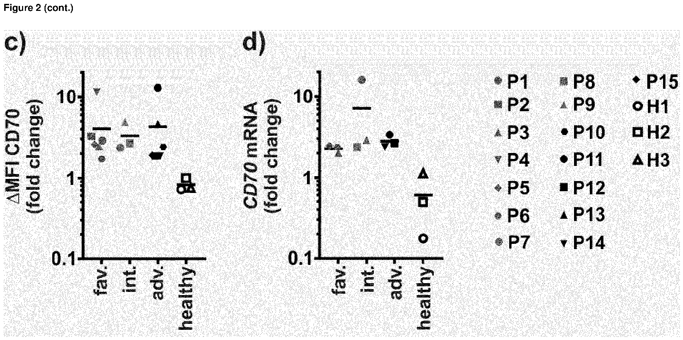

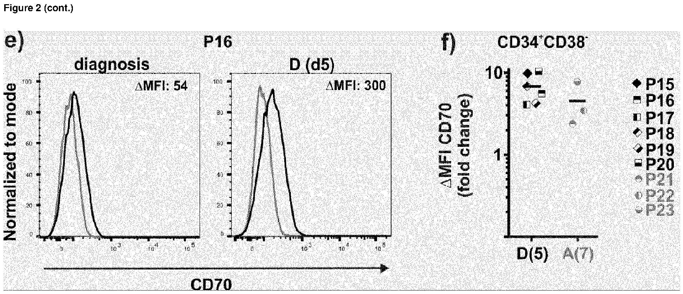

- Figure 2: HMA treatment induces CD70 expression in primary AML stem/progenitor cells. (a) Representative FACS plots of CD70 expression on lin-CD90+CD34+CD38- AML stem/progenitor cells after culture in the presence or absence of 0.5 mM decitabine (D) or vehicle (Veh). Isotype: grey; CD70: black. (b) Cell Viability. (c) Fold change ΔMFI CD70 and (d) mRNA CD70 expression (AML: n=9-15; healthy: n=3). (e) Representative FACS plot and (f) fold change ΔMFI CD70 on stem/progenitor cells in the peripheral blood of AML patients at diagnosis and after 1 cycle of decitabine or azacitidine treatment (D(5), 20 mg/kg, daily for 5 days; A(7), 75 mg/m², daily for 7 days).

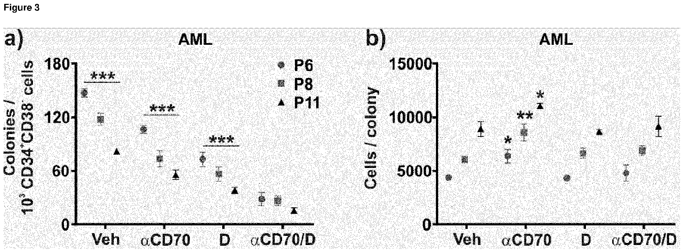

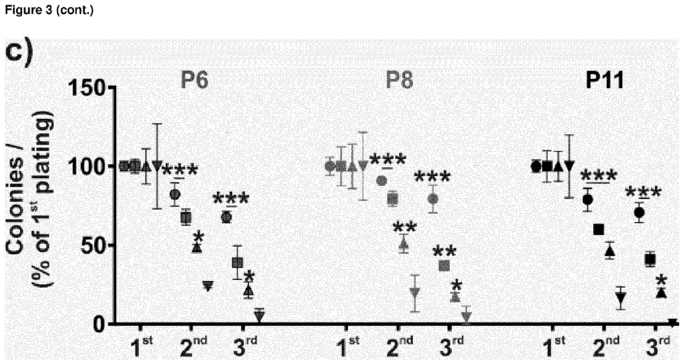

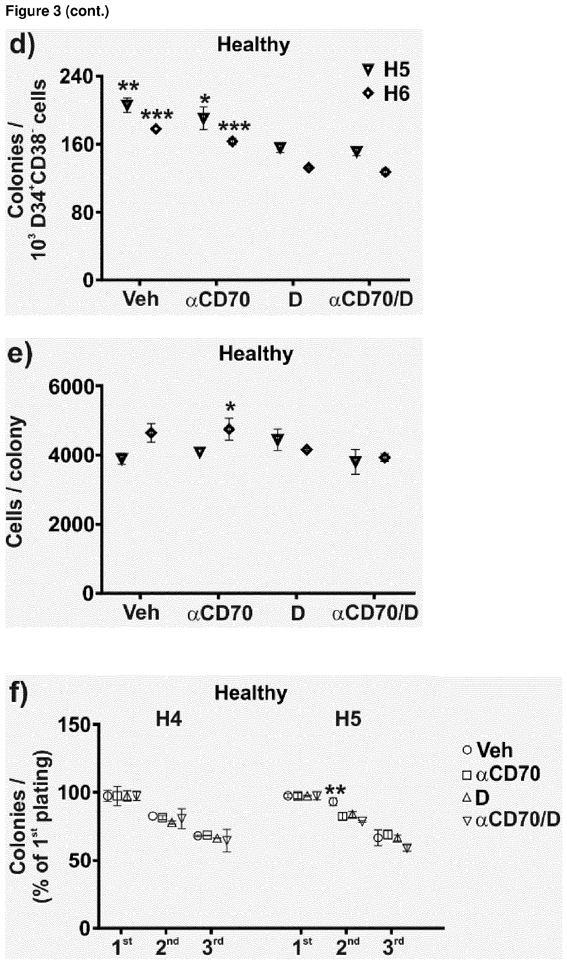

- Figure 3: aCD70/decitabine co-treatment reduces re-plating capacity of human CD34+CD38-AML stem/progenitor cells. (a-b) FACS-purified lin-CD90-CD34+CD38-stem/progenitor cells from BM of newly diagnosed AML patients (P6, P8, P11) were cultured overnight in triplicates in the presence or absence of 10mg/ml anti-CD70 (aCD70) mAb or 0.5 mM decitabine alone or in combination followed by plating into methylcellulose containing aCD70 and decitabine or both. Colonies and cells were enumerated after 14 days. (a) Colonies per 1×10³ plated cells. (b) Cells per colony. (c) Serial re-plating experiments. (d-f) FACS-purified lin-CD90+ CD34+CD38-stem/progenitor cells from BM of "healthy" donors were cultured and plated in methylcellulose as described in (a-c). (d) Colonies per 1×10³ plated cells. (e) Cells per colony. (f) Serial re-plating experiments. Data are represented as mean ± S.D. Statistics: One-way-ANOVA. Dunnett's post-test (vs. aCD70/D); *, P<0.05; **, P<0.01; ***, P<0.001.

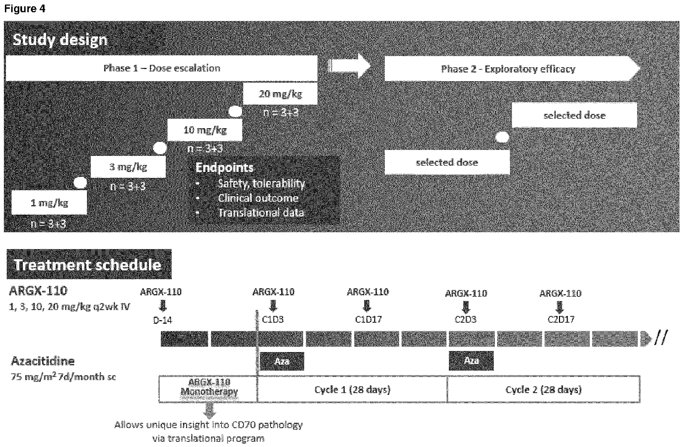

- Figure 4: Schematic showing treatment regimen for patients enrolled in an open-label, dose-escalating study with a proof of concept cohort, of ARGX-110 in combination with AZA.

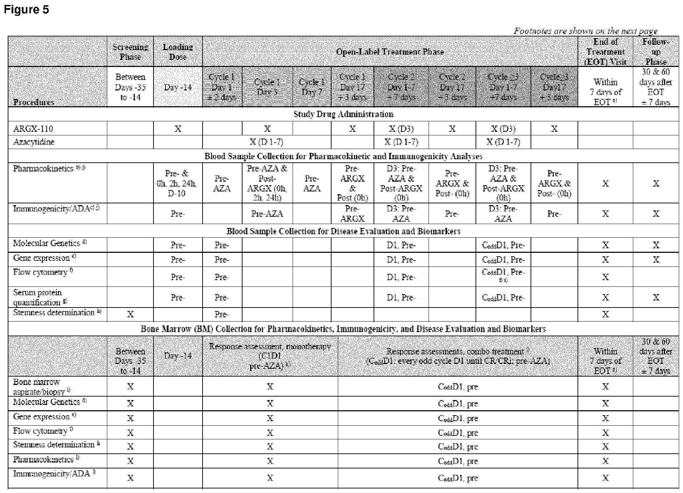

- Figure 5: (a) End-of-treatment (EOT): the EOT date is the last date at which ARGX-110 is administered. A visit is planned within 7 days after the EOT. (b) Time windows are as follows: - at pre-dose time point: up to 4 hours prior to ARGX-110 infusion (Day -14 and Day 17 in each cycle) or administration of AZA (Days 1, 3 and 7) - at 0h (at the end of ARGX-110 infusion) ±30 minutes and at 2h (after end of ARGX-110 infusion): ±30 minutes - at 24h: ±4 hours - at postdose time point (Day X): within 2 hours after the end of ARGX-110 infusion; (c) Up to 4 hours prior to ARGX-110 infusion on Day - 14, and on Day 3 and Day 17 in Cycles 1-4 and in Cycle 8 (if applicable) and at the EOT and follow up visits; (d) Molecular genetics sample may be used for characterization of CD70 and CD11a promoter methylation and for genomic DNA analyses of treatment effect on disease and target pathology. As of cycle ≥3, the sampling should be done before AZA administration at every odd cycle at Day 1 (i.e. C3D1, C5D1, ...) until complete remission (CR, CRi); (e) Gene expression sample will be used for characterization of mRNA levels of CD70, disease and drug effect markers. As of cycle ≥3, the sampling should be done before AZA administration at every odd cycle at Day 1 (i.e. C3D1, C5D1, ...) until complete remission (CR, CRi); (f) Flow cytometry (FACS) sample may be used for additional characterization of minimum residual disease analysis, CD70 and CD27 expression, and drug effect (e.g. blast, NK and T-cells). As of cycle ≥3, the sampling should be done before AZA administration at every odd cycle at Day 1 (i.e. C3D1, C5D1, ...) until complete remission (CR, CRi); (g) Serum sample may be used for additional characterization of sCD27, disease and drug effect markers, inflammatory cyctokine analyses. As of cycle ≥3, the sampling should be done before AZA administration at every odd cycle at Day 1 (i.e. C3D1, C5D1, ...) until complete remission (CR, CRi); (h) Stemness determinations will be performed on mononuclear cells purified from blood or bone marrow. Depending on the number of cells that are harvested, readouts can include Numb staining (determine the ratio of asymmetric/symmetric division), cell and *in vivo* studies (evaluate stem cell potential by e.g. CFU methylcellulose colony assays or survival studies of NSG mice injected with patient mononuclear cells); (i) The timing of the aspirates biopsy sampling for response assessment of the combo treatment should be performed at every odd cycle at Day 1 (i.e. C3D1, C5D1, ...) until complete remission (CR, CRi) and at EOT, unless clinically contra-indicated. For patients achieving CR_{MRD-} an additional BM aspirate/biopsy will be harvested not sooner than 4 weeks after CR_{MRD-} to confirm response. Additional aspirate/biopsy samples can be taken as indicated by the treating physician and will be handled as study related samples; (j) If evidence is obtained for decreased serum exposure of ARGX-110 and/or increased ADA upon prolonged treatment, additional PK and/or ADA samples can be obtained; (k) Bone marrow sample to be taken unless medically contraindicated; (I) The bone marrow supernatant sample may be used to evaluate sCD27 concentration and treatment effect on disease and target pathology.

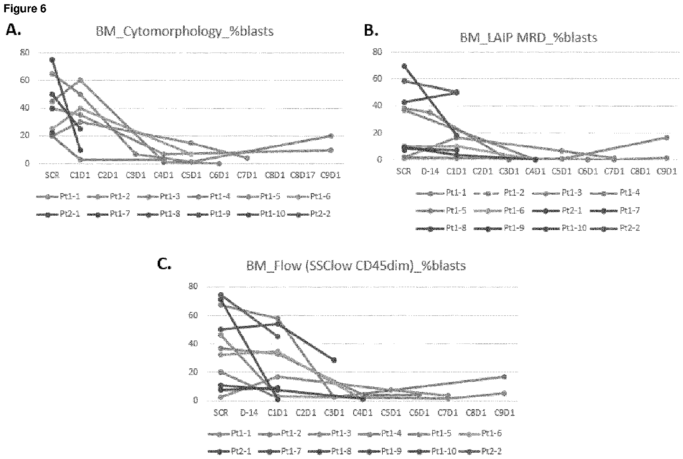

- Figure 6: Bone marrow (BM) blast load was assessed by cytomorphology (A.), and flow cytometry using leukaemia-associated immunophenotype (LAIP) gating (B.) and blast gating (SSClow CD45dim; C.). The latter method was used to determine measurable/minimal residual disease (MRD) status of the patient.

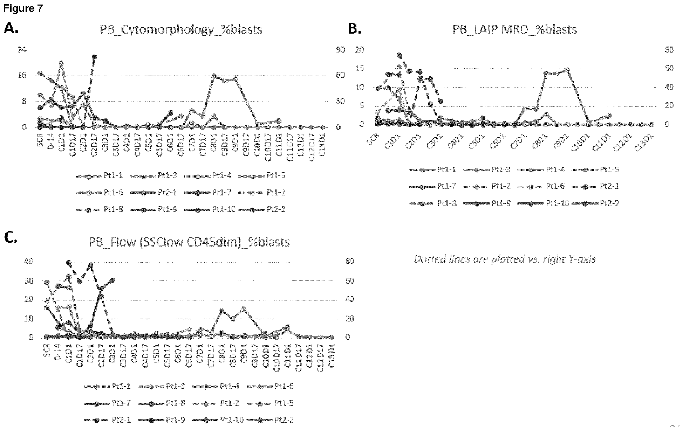

- Figure 7: Peripheral blood (PB) blast load was assessed by cytomorphology (A.), and flow cytometry using leukaemia-associated immunophenotype (LAIP) gating (B.) and blast gating (SSClow CD45 dim; C.). The latter method was used to determine measurable/minimal residual disease (MRD) status of the patient.

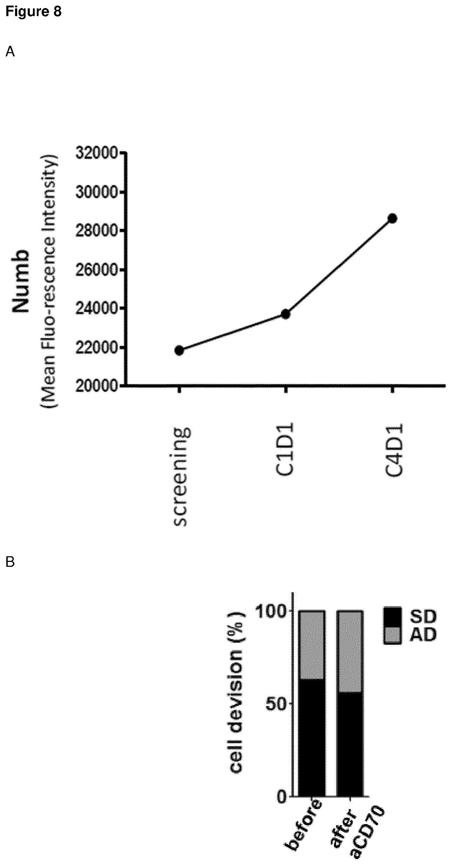

- Figure 8: Purified AML blasts (FACS gate: CD45^{dim} SSC^{low} AV-CD4-CD8-CD19-) were fixed, permeabilized and cells incubated overnight with α-Numb antibody, followed by staining with a fluorescently labeled secondary antibody. DAPI was used to counterstain for DNA. Samples were acquired on an ImageStreamX^{®} Mark II imaging flow cytometer (Amnis / EMD Millipore) and analyzed using INSPIRE^{™} and IDEAS^{®} software (Amnis / EMD Millipore). A.) Total Numb expression (Mean fluorescence intensity) was determined across the slide for bone marrow samples of one patient harvested at baseline ("screening"), after ARGX-110 monotherapy (C1D1) and after combination treatment (C4D1). B.) Symmetrically (SD) and asymmetrically dividing (AD) cells were scored for a bone marrow patient sample at baseline ("before") and after ARGX-110 monotherapy (after αCD70).

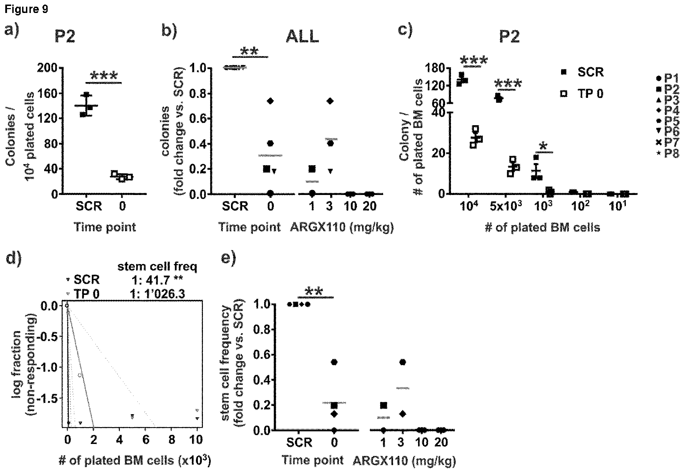

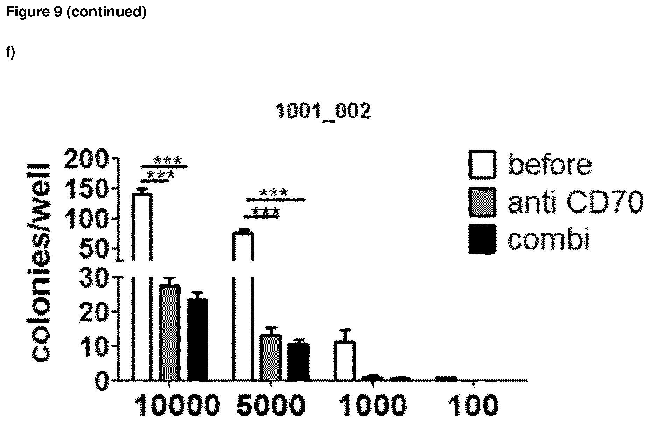

- Figure 9: aCD70 mAb treatment reduces CD34⁺CD38⁻ AML stem/progenitor cell frequencies in AML patients. (a) Colony formation of P2. 10⁴ BM MNCs of patient P002 at diagnosis (SCR) and 14 days after administration of ARGX110 (1mg/kg i.v., time point 0, or C1 D1) were plated in methylcellulose and colony formation was assessed 2 weeks later. (b) Fold change in colony formation of different patients treated with indicated doses of ARGX110. (c) Colony formation at limiting dilution from P2. (d) Stem cell frequencies at SCR and Time point 0 determined in enhanced limiting dilution experiments for P2 and different patients treated with indicated doses of ARGX110. Data are represented as mean ± S.D. Statistics: (a, b, e) Student's t-test; (d): X² test; **, P<0.01; ***, P<0.001. (f) Colony formation of P2. 10⁴, 5 × 10³, 10³ and 10² BM MNCs of patient P002 at diagnosis (SCR), 14 days after administration of ARGX110 (1mg/kg i.v., time point 0, or C1D1) and after combination treatment ("combi") were plated in methylcellulose and colony formation was assessed 2 weeks later.

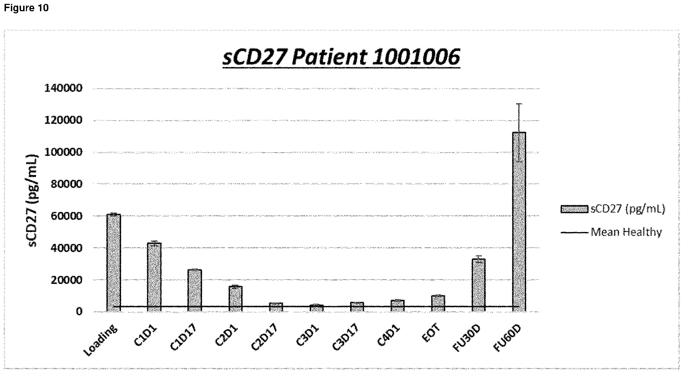

- Figure 10: The serum samples were analysed using an ELISA (Duoset ELISA, human CD27/TNFRSF7 (cat: DY382-05, R&D Systems). Concentration of soluble CD27 in the sample is calculated from a calibration curve. The method allows measurement of the serum concentration of natural human soluble CD27 by means of a sandwich ELISA method. Goat Anti-Human CD27 Capture antibody is coated on a microplate and nonspecific binding sites are blocked. Human serum samples are applied and bound human soluble CD27 is detected and visualized by the subsequent additions of Biotinylated Goat Anti-Human CD27 Detection antibody, Streptavidin-HRP and the mixture of the colour reagent H₂O₂ and the chromogenic substrate tetramethylbenzidine (TMB).

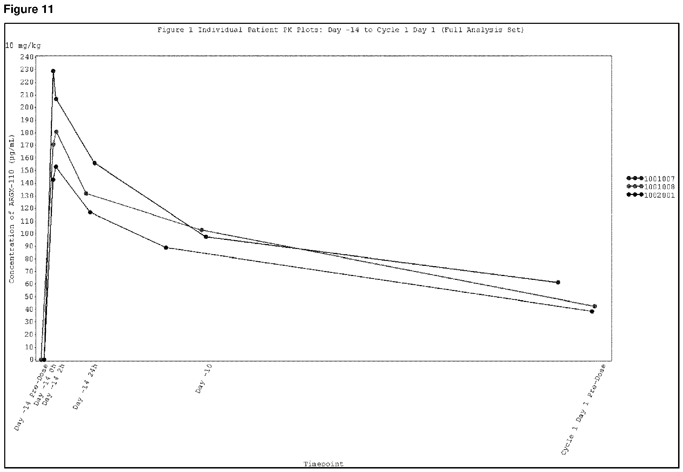

- Figure 11: The serum concentration of ARGX-110 was analyzed using a validated enzymelinked immunosorbent assay (ELISA) method. Individual patient PK plots are provided for 10mg/kg cohort. The PK plot presents data for ARGX-110 cycle1 (D-14 pre-dose until Cycle1 D1 pre-dose).

### DETAILED DESCRIPTION OF INVENTION

Conventional treatment of AML using the so-called "7+3" standard intensive chemotherapy (i.e. intensive doses of cytarabine for 7 days plus 3 days of an anthracycline, typically followed by consolidation chemotherapy or hematopoietic stem cell transplant (HSCT)) has been the standard therapy for many years. However, under this regimen the majority of AML patients under the age of 60 fail to survive more than 5 years. In older adults unfit for standard intensive chemotherapy, outcomes of lower intensity treatment are not curative, HSCT is typically not appropriate, and median overall survival is less than a year. AML is a heterogeneous disease and disease relapse is often due to one or more leukemic stem cell (LSC) clones that were resistant to therapy. Despite numerous preclinical studies investigating alternative therapies, very few have translated into the clinic. There is thus a need for effective new AML therapies, especially those suitable for patients not fit for standard intensive chemotherapy. Ideally, novel therapies not only debulk the disease by reducing blast load in bone marrow and blood but also decrease or even eradicate the heterogeneous LSC populations.

As demonstrated for the first time herein, human subjects having AML or MDS are effectively treated by administration of an anti-CD70 antibody. Such treatment of AML or MDS patients is particularly desirable, as conventional treatment with standard intensive chemotherapy is associated with significant toxicity and side-effects. Furthermore, many patients (for example elderly patients) have comorbidities such that they would not tolerate standard intensive chemotherapy, meaning only less effective therapies are available to them. Provided herein are treatments for AML that are effective even at low doses and with limited side effects and toxicity, meaning they are suitable for administration to all patients. This is especially advantageous for treating patients who otherwise would be ineligible for standard intensive chemotherapy.

Treatment of AML according to the invention therefore provides surprising and significant benefits over currently available therapies. Aspects and embodiments of the invention will now be further described. Unless indicated otherwise or it is technically incompatible, each embodiment of the invention can be taken in combination with any other embodiment of the invention.

### Definitions

Acute myeloid leukaemia (AML) refers to haematopoietic neoplasms involving myeloid cells. AML is characterised by clonal proliferation of myeloid precursors with reduced differentiation capacity. AML patients exhibit an accumulation of blast cells in the bone marrow. Blast cells typically also accumulate in the peripheral blood of AML patients. Typically AML is diagnosed if the patient exhibits 20% or more blast cells in the bone marrow or peripheral blood.

"Blast cells", or simply "blasts", as used herein refers to clonal myeloid progenitor cells exhibiting disrupted differentiation potential. A subset of blast cells is leukemic stem cells (LSCs). These are blast cells having stem cell properties such that, if transplanted into an immuno-deficient recipient, are capable of initiating leukemic disease. LSCs can self-renew by giving rise to leukaemia and also partially differentiate into non-LSC conventional blast cells that resemble the original disease but are unable to self-renew. LSCs occur with a frequency in the range of 1 in 10,000 to 1 in 1 million as a proportion of primary AML blast cells (Pollyea and Jordan, Blood 2017 129:1627-1635, incorporated herein by reference). LSCs may be characterised as cells that are CD34+, CD38-, optionally also CD45- and/or CD123+. LSCs may also be characterised as CD45dim, SSClo, CD90+CD34+ cells.

AML can be categorised and diagnosed according to the WHO 2008 classification, taken in combination with the 2016 update to this classification (Arber et al. Blood, 19 May 2016 vol. 127, no. 20, incorporated herein by reference). According to the WHO classification, AML in general encompasses the following subtypes: acute myeloid leukaemia with recurrent genetic abnormalities; AML with myelodysplasia-related changes; therapy-related myeloid neoplasms; myeloid sarcoma; myeloid proliferations related to Down syndrome; blastic plasmacytoid dendritic cell neoplasm; and AML not otherwise categorized (e.g. acute megakaryoblastic leukaemia, acute basophilic leukaemia).

AML can also be categorised according to the French-American-British (FAB) classification, encompassing the subtypes: M0 (acute myeloblastic leukaemia, minimally differentiated); M1 (acute myeloblastic leukaemia, without maturation); M2 (acute myeloblastic leukaemia, with granulocytic maturation); M3 (promyelocytic, or acute promyelocytic leukaemia (APL)); M4 (acute myelomonocytic leukaemia); M4eo (myelomonocytic together with bone marrow eosinophilia); M5 (acute monoblastic leukaemia (M5a) or acute monocytic leukaemia (M5b)); M6 (acute erythroid leukaemias, including erythroleukaemia (M6a) and very rare pure erythroid leukaemia (M6b)); or M7 (acute megakaryoblastic leukaemia).

As used herein, "AML" refers to any of the conditions encompassed by the WHO and/or FAB classifications, unless specified otherwise. Certain AML subtypes are considered to be of more favourable prognosis, some of intermediate prognosis and some of poor or adverse prognosis. The skilled person is aware of which subtypes would fall into which risk catgory.

Myelodysplastic syndrome (MDS) is characterised by dysplasia, cytopaenia and/or abnormal changes in bone marrow cellurlarity and/or myeloid differentiation, for example increased blast cell infiltration. MDS can be categorised and diagnosed according to the WHO 2008 classification. According to the WHO classification, MDS in general encompasses the following subtypes: MDS with single lineage dysplasia (previously called "refractory cytopenia with unilineage dysplasia", which includes refractory anemia, refractory neutropenia, and refractory thrombocytopenia); MDS with ring sideroblasts, which includes subgroups with single lineage dysplasia and multilineage dysplasia (previously called "refractory anemia with ring sideroblasts"); MDS with multilineage dysplasia (previously called "refractory cytopenia with multilineage dysplasia"); MDS with excess blasts (MDS-EB, previously called "refractory anemia with excess blasts"), which can be further subclassified into MDS-EB-1 and MDS-EB-2 based on blast percentages; MDS with isolated del(5q); and MDS, unclassified.

MDS can also be categorised according to the French-American-British (FAB) classification, encompassing the subtypes: M9980/3 (refractory anaemia (RA)); M9982/3 (refractory anaemia with ring sideroblasts (RARS)); M9983/3 (refractory anaemia with excess blasts (RAEB)); M9984/3 (refractory anaemia with excess blasts in transformation (RAEB-T)); and M9945/3 (chronic myelomonocytic leukaemia (CMML)).

As used herein, "MDS" refers to any of the conditions encompassed by the WHO and/or FAB classifications, unless specified otherwise. For both AML and MDS, the WHO categorisation is preferred herein.

It is particularly desirable to treat "high risk" MDS patients - that is, MDS patients with a high risk of evolving to AML and with a poor survival prognosis. Whether a MDS patient is a "high risk" patient can be determined using the Revised International Prognostic Scoring System for MDS, or IPSS-R (Greenberg et al, Blood. 2012 Sep 20; 120(12): 2454-2465, incorporated herein by reference). The IPSS-R takes into account the patient's bone marrow blast percentage, cytogenetic abnormalities, the number and extent of cytopenias in order to place a patient in a prognostic category. A patient with an IPSS-R score greater than 4.5 is considered to be a "high-risk" MDS patient.

A subject's response to AML therapy can be can be clinically characterised according to the criteria of Table 1:

**Table 1**

| **Response Criteria** | **Definition** |
|---|---|
| Complete remission (CR)* | Bone marrow blasts < 5%; absence of blasts with Auer rods; absence of extramedullary disease; absolute neutrophil count > 1.0 x 10⁹/L (1000/µL); platelet count > 100 x 10⁹/L (100.000/µL); independence of red cell transfusions |
| CR with incomplete recovery (CRi) | All CR criteria except for residual neutropenia (< 1.0 x 10⁹/L [1000/µL]) or thrombocytopenia (< 100 x 10⁹/L [100.000/µL]) |
| Morphologic leukemia-free state (MLFS) | Bone marrow blasts < 5%; absence of blasts with Auer rods; absence of extramedullary disease; no hematologic recovery required |
| Partial remission (PR) | all hematologic criteria of CR; decrease of bone marrow blast percentage to 5% to 25%; and decrease of pretreatment bone marrow blast percentage by at least 50% |

As used herein, "dose" is taken to mean an effective amount of an active agent. Administration of a dose to a subject is the administration of an effective amount of a particular active agent per day. The "dose" may be administered as a single administration, or as multiple administrations per day, provided the indicated effective amount of the dose is administered to the subject. For example, a dose of 1mg/kg can be administered as one daily administration of 1mg/kg, or two administrations per day, the two administrations reaching a total of 1mg/kg.

As used herein, the terms "CD70 protein" or "CD70 antigen" or "CD70" or "TNFSF7" or "CD27L" are used interchangeably and refer to a member of the human TNF ligand family which is a ligand for TNFRSF27/CD27. Specific examples of human CD70 include the polypeptide having the amino acid sequence shown under NCBI Reference Sequence Accession No. NP_001243, or the extracellular domain thereof.

As used herein, the term "antibody" includes an immunoglobulin having a combination of two heavy and two light chains which have significant specific immuno-reactive activity to an antigen of interest (e.g. human CD70). The term "CD70 antibodies" is used herein to refer to antibodies which exhibit immunological specificity for human CD70 protein. "Specificity" for human CD70 does not exclude cross-reaction with species homologues of CD70. Antibodies comprise light and heavy chains, with or without an interchain covalent linkage between them. An antigen-binding fragment of an antibody includes peptide fragments that exhibit specific immuno-reactive activity to the same antigen as the antibody (e.g. CD70). Examples of antigen-binding fragments include: an antibody light chain variable domain (VL); an antibody heavy chain variable domain; a single chain variable fragment or single chain antibody (scFv); a Fab fragment; a F(ab')₂ fragment; a Fd fragment; a Fv fragment; a one armed (monovalent) antibody; diabodies; triabodies; tetrabodies; or any antigen binding molecule formed by combination, assembly or conjugation of such antigen binding fragments. Fragments can be obtained, for example, by chemical or enzymatic treatment of an intact or complete antibody or antibody chain, or by recombinant means.

As used herein, "nucleoside metabolic inhibitors" (NMIs) refers to molecules which interfere with epigenetic modification (e.g. methylation, demethylation, acetylation, or deacetylation) of nucleotides (DNA and/or RNA). Examples of nucleoside metabolic inhibitors include hypomethylating agents (HMAs), isocitrate dehydrogenase (IDH) inhibitors, histone deacetylase (HDAC) inhibitors, and bromodomain and extraterminal (BET) inhibitors. Preferred nucleoside metabolic inhibitors are hypomethylating agents. Hypomethylating agents inhibit normal methylation of DNA and/or RNA. Examples of hypomethylating agents are azacitidine, decitabine and guadecitabine.

As used herein, where two or more active agents are administered as a "combination therapy" this does not require or exclude that the active agents are administered simultaneously or are formulated into a single composition. A combination therapy is given its conventional interpretation of two or more active agents administered such that the patient can derive a benefit from each agent. For the avoidance of doubt, "combination therapy" does not necessitate co-administration, simultaneous administration or fixed dose formulation.

As used herein, "standard intensive chemotherapy" refers to the so-called "7+3" induction chemotherapy characterised by 7 days of high dose cytarabine followed by 3 days of anthracycline administration (e.g. daunorubicin or idarubicin). Intensive chemotherapy is given with the aim of inducing complete remission of AML, typically with the intention of the patient undergoing a stem cell transplant following successful chemotherapy.

Standard intensive chemotherapy is associated with significant toxicity and side-effects, meaning it is not suitable for patients unable to tolerate these effects. As used herein, these patients are termed "ineligible for standard intensive chemotherapy". A patient may be ineligible for standard intensive chemotherapy because, for example, they exhibit one or more comorbidities indicating they would not tolerate the toxicity, or the prognostic factors characterising their disease indicate an unfavourable outcome of standard intensive chemotherapy. Determination of an individual patient's eligibility for standard intensive chemotherapy would be performed by a clinician taking into account the individual patient's medical history and clinical guidelines (e.g. the National Comprehensive Cancer Network (NCCN) guidelines, incorporated herein by reference). AML patients over the age of 60 are often assessed as ineligible for standard intensive chemotherapy, with other factors to be considered including the cytogenetics and/or molecular abnormalities of the AML being treated.

A patient ineligible for standard intensive chemotherapy may instead receive chemotherapy of reduced intensity, such as low dose cytarabine (LDAC). Patients ineligible for standard intensive chemotherapy and for whom LDAC is not appropriate can receive best supportive care (BSC), including hydroxyurea (HU) and transfusion support.

As used herein, "subject" and "patient" are used interchangeably to refer to a human individual.

### Detailed Description

As demonstrated herein, patients suffering from myeloid neoplasms such as AML and MDS can be treated with an anti-CD70 antibody. Following a single administration of anti-CD70 antibody, the number of leukemic stem cells able to be isolated from subjects' bone marrow was significantly reduced, as was the number of blast cells detected in the bone marrow and peripheral blood. This result was observed even at surprisingly low doses of the antibody.

Therefore, in a first aspect the invention provides a method for treating acute myeloid leukaemia (AML) or myelodysplastic syndrome (MDS) in a subject comprising administering to the subject one or more doses of an anti-CD70 antibody or antigen-binding fragment thereof. The invention provides a method for treating acute myeloid leukaemia (AML) or myelodysplastic syndrome (MDS) in a subject comprising administering to the subject one or more therapeutically effective doses of an anti-CD70 antibody or antigen-binding fragment thereof.

In a further aspect is provided a method for reducing the percentage of blasts in the bone marrow and/or peripheral blood of a subject with AML or MDS, the method comprising administering to the subject one or more therapeutically effective doses of an anti-CD70 antibody or antigen-binding fragment thereof.

In a further aspect is provided an anti-CD70 antibody or antigen-binding fragment thereof for use in the methods provided herein.

*Anti-CD70 antibody monotherapy and aCD70+NMI combination therapy reduce bone marrow and peripheral blood blast cells*

As described herein, methods of the invention reduce the number and/or proportion of blast cells in the bone marrow and/or in the peripheral blood, preferably in both the bone marrow and peripheral blood.

The proportion of blast cells in the bone marrow or peripheral blood can be assessed by methods known in the art and described herein, for example flow cytometric or cell morphologic assessment of cells obtained from a bone marrow biopsy of the subject, or a peripheral blood smear. The proportion of blasts is determined versus total cells in the sample. For example, flow cytometry can be used to determine the proportion of blast cells using the number of CD45^{dim}, SSC^{low} cells relative to total cell number. By way of further example, cell morphological assessment can be used to determine the number of morphologically identified blasts relative to the total number of cells in the field of view being examined.

In certain embodiments are provided methods for reducing the proportion of blasts cells in the bone marrow by at least 5% in absolute terms - that is, reducing the percentage of blast cells in the bone marrow from 30% to 25% or less, for example. In certain embodiments, treatment can be characterised as reducing the proportion of blasts cells in the bone marrow by at least 10% in absolute terms, preferably at least 15%, preferably at least 20%, preferably at least 25%, preferably at least 30%, preferably at least 40%, preferably at least 50%, preferably at least 55%, preferably at least 60% in absolute terms. In certain embodiments, the proportion of blasts cells in the bone marrow is reduced by at least 50% compared to before treatment.

In certain embodiments, the proportion of bone marrow blasts is measured by cell morphological assessment. In certain embodiments, the proportion of bone marrow blasts is measured by flow cytometric assessment. In certain embodiments, the proportion of bone marrow blasts is measured in accordance with minimum residual disease (MRD) assessment.

In certain embodiments are provided methods for reducing the proportion of blasts cells in the peripheral blood by at least 5% in absolute terms - that is, reducing the percentage of blast cells in the peripheral blood from 30% to 25% or less, for example. In certain embodiments are provided treatments for reducing the proportion of blasts cells in the peripheral blood by at least 5% in absolute terms, optionally at least 10% in absolute terms, preferably at least 15%, preferably at least 20%, preferably at least 25% in absolute terms. In certain embodiments, the proportion of peripheral blood (PB) blasts is measured by cell morphological assessment. In certain embodiments, the proportion of PB blasts is measured by flow cytometric assessment. In certain embodiments, the proportion of PB blasts is measured in accordance with minimum residual disease (MRD) assessment.

In certain embodiments are provided methods for reducing the proportion of blasts cells in the bone marrow to less than 40%, optionally to less than 20%, for example less than 10%. In certain embodiments are provided methods for reducing the proportion of blasts cells in the bone marrow to less than 5%.

In certain embodiments are provided methods for reducing the proportion of blasts cells in the peripheral blood to less than 40%, optionally less than 20%, for example less than 10%. In certain embodiments are provided methods for reducing the proportion of blasts cells in the peripheral blood to less than 5%.

In certain embodiments, the subject has a bone marrow blast percentage of at least 20%, optionally at least 40%, optionally at least 60%, optionally at least 70%, prior to treatment.

In certain embodiments, the subject has a peripheral blood blast percentage of at least 5% prior to treatment, optionally of at least 8%, optionally of at least 10%, optionally of at least 15%, optionally of at least 20%, optionally of at least 30%, optionally of at least 40%, optionally of at least 50%, optionally at least 60% prior to treatment.

For clinical determination of blast cell percentage, typically cell morphological (also known as cytomorphology) assessment is preferred.

*Anti-CD70 antibody monotherapy and aCD70+NMI combination therapy reduce the level of leukaemic stem cells and promote differentiation*

An important subset of blasts in AML is the leukemic stem cells (LSCs). LSCs are cancer stem cells capable of initiating leukemic disease. LSCs can self-renew by giving rise to leukaemia and also partially differentiate into non-LSC conventional blast cells that resemble the original disease but are unable to self-renew. LSCs may be characterised as cells that are CD34+, CD38-, optionally also CD45- and/or CD123+. LSCs may also be characterised as CD45dim, SSClo, lin-CD90+CD34+ cells. Reducing the number of LSCs in an AML patient should greatly improve the prospect of remission and reduce the potential for relapse.

As demonstrated herein, the monotherapy and combination treatments according to the invention reduce the proportion of LSCs in a patient by at least 2-fold and in some instances to a much greater extent. Treatment according to the invention is therefore expected to not only reduce the overall blast percentage in a patient, but also reduce the likelihood of subsequent relapse of disease.

Therefore, in certain embodiments the methods of the invention reduce the number of LSCs as a proportion of mononuclear cells (also referred to as a reduction in LSC frequency). In certain embodiments are provided methods for reducing LSC frequency by at least a factor of 2.

In certain embodiments, assessment of the proportion of LSCs in the bone marrow can be determined by serial dilution plating on e.g. methylcellulose.

Monotherapy and combination treatments according to the invention also promote LSC differentiation into myeloid cells. Promotion of such differentiation reduces the population of LSCs able to self-renew, thereby increasing the prospect of remission and reducing the risk of relapse.

Myeloid differentiation of LSCs is characterised by asymmetric cell division, in contrast to symmetric cell division which results in two daughter stem cells. Asymmetric division can be assessed via microscopy techniques or by measuring cell fate determination markers such as the Numb protein. As demonstrated herein, LSCs from patients which have received anti-CD70 antibody monotherapy exhibit increased Numb expression (and therefore increased differentiation) compared to before treatment. Numb expression (and therefore differentiation) is further increased when patients are treated with anti-CD70 antibody in combination with an NMI. These data represent the first clinical demonstration of an anti-CD70 antibody increasing differentiation of LSCs *in vivo,* and thereby advance the *in vitro* data reported in Riether etal. J. Exp. Med. 2017 Feb;214(2):359-380.

In certain embodiments, methods according to the invention promote leukaemic stem cell differentiation. In certain embodiments, methods according to the invention promote asymmetric division of leukaemic stem cells. In certain embodiments, methods according to the invention promote expression of Numb on leukaemic stem cells.

*AML is effectively treated even at low doses of anti-CD70 antibody*

The data from the clinical trial presented herein demonstrates that blast cell percentages were reduced in patients being treated with CD70 antibodies even at the lowest dose. The intention of administering the antibodies to patients at this dose was simply to confirm the safety of the antibody treatment and to identify the dose limiting toxicity (DLT). It was therefore highly surprising that even after a single administration of the lowest dose of antibody blast cell percentages were greatly reduced.

The data presented herein therefore demonstrates that AML or MDS can be treated with all tested doses of anti-CD70 antibody, including unexpectedly low doses.

Therefore, in certain embodiments of all methods of the invention the anti-CD70 antibody or antigen-binding fragment thereof is administered at a dose in the range from 0.1 mg/kg to 25 mg/kg per dose, for example in the range of from 0.1 mg/kg to 20 mg/kg. In certain embodiments, the anti-CD70 antibody or antigen-binding fragment thereof is administered at a dose in the range from 1 mg/kg to 20 mg/kg per dose. Ranges described herein include the end points of the range unless indicated otherwise - for example, administration at a dose in the range of 0.1-25 mg/kg includes administration at a dose of 0.1 mg/kg and administration at a dose of 25 mg/kg, as well as all doses between the two end points).

In certain embodiments of methods of the invention, the anti-CD70 antibody or antigen-binding fragment thereof is administered at a dose in the range from 0.1-15 mg/kg. In certain embodiments the anti-CD70 antibody or antigen-binding fragment thereof is administered at a dose in the range from 0.5-2 mg/kg. In certain embodiments the anti-CD70 antibody or antigen-binding fragment thereof is administered at a dose of 1 mg/kg, 3 mg/kg, 10 mg/kg, or 20 mg/kg. In certain preferred embodiments the anti-CD70 antibody or antigen-binding fragment thereof is administered at a dose of 1mg/kg. In certain preferred embodiments the anti-CD70 antibody or antigen-binding fragment thereof is administered at a dose of 10 mg/kg.

In certain embodiments multiple doses of the CD70 antibody or antigen binding fragment are administered. In certain such embodiments, each dose of the anti-CD70 antibody or antigen-binding fragment thereof is separated by 10-20 days, optionally 12-18 days. In certain embodiments each dose of anti-CD70 antibody is separated by 14-17 days.

In certain embodiments, treatment according to the invention results in at least partial remission. In certain embodiments, treatment results in the patient exhibiting a morphologic leukaemia-free state. In certain embodiments, the treatment results in at least complete remission with incomplete recovery (CRi). In certain embodiments, the treatment results in at least complete remission.

The treatment according to the invention is thus able to treat AML or MDS by reducing the number of blast cells (including leukemic stem cells) in the bone marrow and in the peripheral blood. Treatment is also particularly effective at reducing the level of LSCs specifically.

### Patient characteristics

Treatment according to the invention is particularly advantageous as it provides an effective means of inducing remission (partial or complete) of AML without the significant toxicity and morbidity associated with standard intensive chemotherapy. Providing a therapy without these side effects is a significant advantage of the invention.

Reducing the side effects of AML therapy is clearly desirable for the treatment of AML patients in general. In addition, it is particularly advantageous for patients who would not ordinarily be able to receive the more potent AML treatments. There is a need for an effective therapy for patients ineligible for standard intensive chemotherapy due to comorbidities of the patient, for example that they are 60 years old or older. Whilst alternative therapies for these patients are available (e.g. low dose cytarabine (LDAC)), they provide a reduced likelihood of achieving remission and are still associated with significant side-effects. In contrast, treatment according to the invention provides an effective therapy for AML that can be tolerated by patients who would not be able to tolerate the toxicity of standard intensive chemotherapy.

Therefore, in certain embodiments of the invention, the subject is not eligible for standard intensive chemotherapy prior to treatment. In certain such embodiments, the subject is at least 60 years old, optionally at least 70 years old.

A further conventional therapy for AML is haematopoietic stem cell transplant (HSCT), using either allogenic or autologous stem cells. Patients ineligible to receive standard intensive chemotherapy are typically precluded from receiving HSCT. This is because the prospect of successful transplant is reduced in patients receiving only low dose chemotherapy, since they are unable to receive the standard intensive chemotherapy intended to ablate the bone marrow blasts in preparation for receiving the transplant. Whilst HSCT can be conducted using reduced intensity chemotherapy, the prospect of success is lower due to the greater likelihood of residual blasts remaining in the bone marrow.

The suitability of an individual patient for HSCT will depend on the case by case assessment of a clinician, taking into account the prospect of the transplant being successful and other factors such as remaining treatment options and quality of life. An exemplary but significant factor to consider is the percentage of bone marrow blasts exhibited by the patient.

As demonstrated herein, treatment according to the invention is able to significantly reduce the percentage of bone marrow blasts. By providing a means for reducing the bone marrow blast percentage in patients who are ineligible for standard intensive chemotherapy, the methods of the invention open up the prospect of a successful HSCT in a population of patients for whom HSCT was not previously recommended.

Therefore in certain embodiments of all aspects of the invention, the subject is not eligible for standard intensive chemotherapy prior to treatment and the treatment reduces the percentage of bone marrow blasts such that the subject is eligible for HSCT. In certain embodiments the treatment further comprises conducting a haematopoietic stem cell transplant on the subject.

Provided herein is a method for preparing a subject with AML or MDS for haematopoietic stem cell transplant (HSCT) comprising administering to the subject one or more doses of an anti-CD70 antibody or antigen-binding fragment thereof.

In certain embodiments and as described elsewhere herein, the method may further comprise administering a NMI (for example an HMA such as azacitidine) to the subject.

In certain embodiments the method further comprises conducting a HSCT on the subject.

Treatment according to the invention is expected to be particularly effective at treating patients with abnormally high levels of soluble CD27 in serum.

Without wishing to be bound by theory, this is thought to be because CD27-CD70 binding results in the release of soluble CD27 (sCD27) by shedding. Soluble CD27 can therefore serve as a biomarker for the extent of CD70/CD27 interactions (Riether et al. J. Exp. Med. 2017 Feb;214(2):359-380, incorporated herein by reference). In particular, sCD27 is thought to correlate with the percentage of blast cells in bone marrow, as well as to serve as a marker for the sternness of a patient's blasts, with increased sCD27 indicating increased levels of stemness (Riether et al. J. Exp. Med. 2017 Feb;214(2):359-380).

CD70/CD27-mediated signalling is thought to promote aberrant cell divisions and high levels of serum sCD27 are correlated with poor prognosis of AML patients. Use of an anti-CD70 antibody in accordance with the invention reduces the percentage of blast cells in bone marrow (see Examples) and blocks CD27/CD70 interactions, thereby decreasing levels of blast stemness and promoting differentiation. Accordingly, AML patients exhibiting elevated sCD27 are expected to derive particular benefit from treatment with anti-CD70 antibody according to the invention.

Selected healthy individuals' samples exhibit levels of sCD27 in serum of between 10 and 200 U/ml, with AML patients exhibiting elevated serum sCD27 (Riether et al. J. Exp. Med. 2017 Feb;214(2):359-380). Therefore, in certain embodiments of the invention, the patient to be treated has a serum sCD27 concentration of greater than 200 U/ml. It has also been determined that, for all AML patients across ages and AML sub-type risk categories (that is, across patients with favourable, intermediate and high risk/adverse cytogenetic classifications) those with a serum sCD27 level greater than a threshold of 577 U/ml have a poorer prognosis than those patients with sCD27 levels lower than this threshold. Therefore in certain embodiments, the patient to be treated has a serum sCD27 concentration of greater than 577 U/ml.

When serum sCD27 levels were analysed within AML subtype risk categories, it was identified that patients with AML of a favourable subtype having serum sCD27 level greater than a threshold of 470 U/ml have a poorer prognosis than patients with AML of favourable subtype and with sCD27 levels lower than this threshold. Therefore in certain embodiments, the patient to be treated has a favourable or low risk AML subtype and has a serum sCD27 concentration of greater than 470 U/ml.

For patients with AML of an intermediate risk subtype, those having serum sCD27 level greater than a threshold of 586 U/ml have a poorer prognosis than patients with AML of intermediate subtype and with sCD27 levels lower than this threshold. Therefore in certain embodiments, the patient to be treated has an intermediate risk AML subtype and has a serum sCD27 concentration of greater than 586 U/ml.

For patients with AML of a high risk subtype, those having serum sCD27 level greater than a threshold of 714 U/ml have a poorer prognosis than patients with AML of high risk subtype and with sCD27 levels lower than this threshold. Therefore in certain embodiments, the patient to be treated has a high risk AML subtype and has a serum sCD27 concentration of greater than 714 U/ml.

It will be apparent that a patient to be treated according to the invention can have had their serum sCD27 concentration previously determined as being above or below any of the described thresholds. Nevertheless, in certain embodiments, the treatment and methods of the invention further comprise the step of measuring the patient's serum CD27 concentration.

As already described herein, administration of an anti-CD70 antibody in accordance with the invention reduces the number of bone marrow blasts and can block the interaction of CD27 and CD70. Reduction of blast cells alongside inhibition of CD27/CD70 interaction significantly reduces the levels of sCD27 shedding, as demonstrated in the Examples.

In certain embodiments, methods according to the invention (for example monotherapy with anti-CD70 antibody or a combination therapy of anti-CD70 antibody+NMI) reduce serum sCD27 compared to before treatment. In certain embodiments, serum sCD27 is reduced by at least 200 pg/ml, optionally at least 500 pg/ml. In certain embodiments, serum CD27 is reduced by at least 1000 pg/ml, at least 1500 pg/ml or at least 10,000 pg/ml compared to before treatment.

It is further demonstrated in the Examples herein that levels of sCD27 could be used as a correlate of anti-CD70 antibody engagement of its target antigen. Soluble CD27 levels can decrease in response to CD70 antibody administration and then subsequently increase when therapy is stopped. Accordingly, in some embodiments the methods further comprising monitoring treatment efficacy by detecting serum sCD27.

Administration of an anti-CD70 antibody in accordance with the invention not only reduces the percentage of blasts but preferentially reduces the number of blast cells that express CD70 (CD70+ blasts). Aberrant AML blast cells are understood to overexpress CD70 relative to healthy progenitor cells. Targeting CD70-expressing blast cells thus reduces the number of pathogenic blasts but with a negligible effect on healthy progenitor cells, thereby reducing the risk of the patient developing cytopenias.

In certain embodiments of the invention the percentage of CD70+ blasts is reduced. In certain embodiments the percentage of CD70+ blasts is reduced by at least 5%, optionally by at least 10% in absolute terms. In certain embodiments, the percentage of CD70+ cells is reduced to less than 20%, optionally less than 10%. In certain embodiments the percentage of CD70+ blast cells is reduced to less than 5%. The percentage of CD70+ blasts may be reduced by the indicated amount in the bone marrow and/or the peripheral blood.

Regarding treatment of MDS, it is particularly desirable to treat "high risk" MDS patients - that is, MDS patients with a poor survival prognosis, faster disease progression and and a higher probability of progressing to AML. Therefore, in certain embodiments, the patient is a "high risk" MDS patient. In certain embodiments, the patient has an IPSS-R score greater than 4.5.

### Anti-CD70 antibody therapy in combination with a nucleoside metabolic inhibitor (NMI)

As already described herein, monotherapy with anti-CD70 antibody provides an effective treatment for AML or MDS, resulting in significant reduction of blast cell percentage. As well as providing an effective monotherapy, the data herein demonstrates further therapeutic efficacy of anti-CD70 antibodies when administered as part of a combination therapy with a nucleoside metabolic inhibitor (NMI), for example a hypomethylating agent (HMA) such as such azacitidine (also referred to herein as azacytidine, AZA or aza) or decitabine.

Thus, in a further aspect is provided a combination comprising an anti-CD70 antibody or antigen binding fragment thereof and a NMI. In certain embodiments, the NMI is a hypomethylating agent, preferably azacitdine.

In a further aspect is provided a combination comprising an anti-CD70 antibody or antigen binding fragment thereof and a NMI for use in a method of treating AML or MDS. In certain embodiments, the NMI is a hypomethylating agent, preferably azacitidine.

In a further aspect is provided a combination comprising an anti-CD70 antibody or antigen binding fragment thereof and a NMI for use in a method of the present invention. In certain embodiments, the NMI is a hypomethylating agent, preferably azacitidine.

The following embodiments are applicable to all aspects and methods of the invention provided herein unless specified otherwise.

Azacitidine is an analogue of cytidine and decitabine is its deoxy derivative. AZA and decitabine are inhibitors of DNA methyltransferases (DNMT) known to upregulate gene expression by promoter hypomethylation. Such hypomethylation disrupts cell function, thereby resulting in cytotoxic effects.

Without wishing to be bound by theory, the therapeutic effect arising from treatment with both a CD70 antibody and a nucleoside metabolic inhibitor such as AZA is thought to be due to an enhanced effect from combining the two active agents. As already described, the anti-CD70 antibody alone depletes bone marrow and circulating blasts (i.e. those in bone marrow, the peripheral blood, or both), and a nucleoside metabolic inhibitor (such as AZA) alone disrupts cell activity.

In addition, upregulation of CD70 on the surface of AML blasts and LSCs is induced by treatment with a nucleoside metabolic inhibitor (azacitidine or decitabine)(see Examples and Figure 1). This effect of upregulating the antigen correlates with an increase in the efficacy of the anti-CD70 antibody when used in combination with a nucleoside metabolic inhibitor such as azacitidine, as demonstrated in the accompanying Examples. This results in the number of blast cells being reduced further than after administration of either active agent alone and could explain the surprising efficacy of the combination treatment when anti-CD70 antibody is administered as a low dose.

Therefore, in certain preferred embodiments the treatments and methods of the invention further comprise administering a nucleoside metabolic inhibitor to the subject. In certain embodiments the nucleoside metabolic inhibitor is a hypomethylating agent. In certain embodiments the nucleoside metabolic inhibitor is azacitidine or decitabine. In certain preferred embodiments the nucleoside metabolic inhibitor is azacitidine.

In certain embodiments the nucleoside metabolic inhibitor (e.g. azacitidine) is administered at a dose in the range of 50-100 mg/m² per day. As already noted, ranges described herein include the end points of the range unless indicated otherwise - for example, administration at a dose in the range of 50-100 mg/m² per day includes administration at a dose of 50 mg/m² per day and administration at a dose of 100 mg/m² per day, as well as all doses between the two end points. In certain embodiments, the nucleoside metabolic inhibitor is administered at a dose in the range of 70-80 mg/m² per day. In certain preferred embodiments the nucleoside metabolic inhibitor is administered at a dose of 75 mg/m² per day.

In certain embodiments the nucleoside metabolic inhibitor is administered over a dosing period of a daily dose for 5-9 days. That is, a dose of the nucleoside inhibitor is administered every day for a period 5, 6, 7, 8, or 9 days in length. In certain preferred embodiments the nucleoside metabolic inhibitor is administered over a dosing period of a daily dose for 7 days.

In certain embodiments the nucleoside metabolic inhibitor is administered according to a dosage regimen of repeated dosing periods, wherein the end of one dosing period and the start of the next dosing period are separated by 18-25 days. That is, the dosage regimen includes at least 2 dosing periods in which a dose of the nucleoside inhibitor is administered every day (for example for a period 5, 6, 7, 8, or 9 days in length), wherein the end of the one dosing period and the start of the next dosing period are separated by 18, 19, 20, 21, 22, 23, 24, or 25 days. In certain embodiments the end of one dosing period and the start of the next dosing period are separated 21 days.

In certain embodiments, each dosing period is of the same length (e.g. 7 days). In certain embodiments, the end of each dosing period and the start of the next dosing period are separated by the same number of days (e.g. 21 days).

In certain embodiments, the first dose of nucleoside metabolic inhibitor is administered 7-21 days after the first dose of anti-CD70 antibody or antigen binding fragment thereof. In certain embodiments the first dose of nucleoside metabolic inhibitor is administered 10-17 days after the first dose of anti-CD70 antibody or antigen binding fragment thereof. In certain embodiments the first dose of nucleoside metabolic inhibitor is administered 14 days after the first dose of anti-CD70 antibody or antigen binding fragment thereof.

In certain embodiments one of the daily doses of the nucleoside metabolic inhibitor is administered on the same day as a dose of the anti-CD70 antibody or antigen-binding fragment thereof. That is, in embodiments of the methods of the invention in which the subject is administered both an anti-CD70 antibody (or antigen binding fragment thereof) and a nucleoside metabolic inhibitor, the dosage regimes of both the anti-CD70 antibody and the nucleoside metabolic inhibitor are such that at least one of the scheduled doses of the anti-CD70 antibody is on the same day as one of the scheduled daily doses of the nucleoside metabolic inhibitor. That day could be the first, second, third, fourth, fifth, sixth or seventh day of the dosing period of the nucleoside metabolic inhibitor.

In certain embodiments, a dose of the anti-CD70 antibody or antigen-binding fragment thereof is administered every 14-17 days and the nucleoside metabolic inhibitor is administered according to a dosage regimen of repeated dosing periods of a daily dose for 7 days, wherein the end of one dosing period and the start of the next dosing period are separated by 21 days, and wherein the first daily dose of the first dosing period is administered 14 days after the first dose of the anti-CD70 antibody or antigen-binding fragment thereof.

It is a further advantage of the invention that following an initial period of combination therapy, the administration of the NMI (e.g. aza) can be tapered or stopped. For example, as demonstrated herein, an initial period of combination therapy with CD70 antibody and NMI can significantly reduce the patient's blast percentage. There is however the potential for accumulated toxicity arising from prolonged periods of NMI treatment, for example cytopenias arising from the effect of NMIs on non-blast cell types. By tapering or stopping the dose of NMI after an initial period, the risk of such toxicity will be reduced and non-blast cell types can recover. However the percentage of blasts will still be controlled by maintaining the dose of anti-CD70 antibody. In other words, the patient can be treated with an induction therapy of the combination treatment already described and can then be transitioned to a maintenance therapy comprising anti-CD70 therapy with tapering doses of NMI or simply CD70 antibody as a monotherapy.

Therefore in certain embodiments, treatment according to the invention comprises administering to the patient an anti-CD70 antibody and a NMI as a combination therapy according to any of the embodiments described above in a first stage (induction therapy), and in a subsequent second stage administering to the patient an anti-CD70 antibody and administering a dose of the NM! lower than the dose of NMI administered in the first stage (maintenance therapy). The dose of the NMI in the second stage may be zero - that is, the second stage can involve administration of the anti-CD70 antibody only.

In such embodiments, the dose of CD70 antibody administered in the second stage (i.e. maintenance therapy) is any dose according to the embodiments already described. That is, in certain embodiments the dose is in the range from 0.1 mg/kg to 25 mg/kg, for example 0.1 mg/kg to 20 mg/kg, for example from 1 mg/kg to 20 mg/kg. In certain embodiments the dose is in the range of from 0.1 mg/kg to 15 mg/kg per dose. In certain embodiments the dose is in the range from 0.5 mg/kg to 2 mg/kg. In certain embodiments the dose is 1 mg/kg, 3 mg/kg, 10 mg/kg, or 20 mg/kg. In certain embodiments the dose is 1 mg/kg. In certain embodiments the dose is 10 mg/kg.

The duration of the first stage (i.e. induction therapy), the timing of the transition to the second stage (i.e. maintenance therapy) and the extent to which the dose of NMI is tapered or stopped entirely are factors that will be tailored to the individual patient and determined by their clinician according to the individual patient's response to therapy and their medical history. Therefore the following embodiments are provided by way of non-limiting example.

In certain embodiments the induction therapy is administered to the patient until their bone marrow and/or peripheral blood blast percentage is less than10%, optionally less than 5%. In certain embodiments, the induction therapy is administered for at least 5 NMI dosing periods, optionally at least 6, 7, 8, 9, or at least 10 NMI dosing periods.

In certain embodiments, the dose of the NMI in the maintenance period is no more than 50 mg/m² per day, optionally no more than 40 mg/m² per day, optionally no more than 30 mg/m² per day, optionally no more than 20 mg/m² per day.

### Pharmaceutical compositions

Also provided in accordance with the invention are pharmaceutical compositions for use in the methods described herein. Therefore in a further aspect of the invention is provided a pharmaceutical composition comprising an anti-CD70 antibody and a pharmaceutically acceptable excipient or carrier for use in a method according to the invention. Suitable pharmaceutically acceptable carriers and excipients would be familiar to the skilled person. Examples of pharmaceutically acceptable carriers and excipients suitable for inclusion in pharmaceutical compositions of the invention include sodium citrate, glycine, polysorbate (e.g. polysorbate 80) and saline solution.

For combinations of the invention, the anti-CD70 antibody or antigen binding fragment may be formulated for administration via the same route or via a different route as compared with the nucleoside metabolic inhibitor.

In certain embodiments, the anti-CD70 antibody is administered to the subject parenterally, preferably intravenously (i.v.). In certain embodiments the anti-CD70 antibody is administered as a continuous i.v. infusion until the desired dose is achieved.

In certain embodiments, the nucleoside metabolic inhibitor is administered parenterally, preferably subcutaneously (s.c.).

### Combination therapies

Treatment in accordance with the invention can be incorporated into a combination therapy with one or more additional active agents, such as therapeutic or palliative agents (e.g. radiotherapy, analgesics or antibiotics). Such active agents may be administered as therapeutics, to improve patient's response to therapy and/or their quality of life. Therefore in certain embodiments, the CD70 antibody (optionally in a combination therapy with a NMI) is administered in accordance with the invention in a combination therapy with one or more additional active agents.

Treatment of AML or MDS with an anti-CD70 antibody (as a monotherapy or as a combination therapy with an NMI such as aza) has surprisingly been shown to be particularly effective at reducing blasts in bone marrow and peripheral blood. A particular advantage is that the CD70 antibodies are selective for blasts over other cell types (as CD70 has minimal expression in normal tissues) and can induce stem cell differentiation /reduce their stemness (for example by interfering with CD27/CD70 mediated signalling).

These effects on blasts and leukaemic stem cells indicate that treatment in accordance with the invention will be advantageously combined with agents that also target blasts and LSCs. CD33 is a receptor that is expressed on the majority of myeloid blasts and LSCs but is absent or present only at low levels on normal haematopoietic stem cells. Whilst CD33 is expressed on a variety of other cell types less associated with AML, CD33 is expressed on the majority of AML cells and the level of CD33 seems to correlate with the disease prognosis. Antibodies to CD33 such as bispecific antibody AMG330 and antibody drug conjugate (ADC) vadastuximab talirine have been described as effectively treating AML and vadastuximab talirine (also known as SGN-CD33A from Seattle Genetics) is being tested in phase III trials. It is therefore expected that anti-CD33 antibodies will combine with treatment according to the invention (monotherapy with anti-CD70 antibody or combination of anti-CD70 antibody and NMI (e.g. aza)) to provide a particularly effective therapy for AML. Therefore, in certain embodiments of the invention, the patient is administered an anti-CD33 agent, for example an anti-CD33 antibody, as part of a combination therapy with the anti-CD70 antibody (and optionally NMI) in accordance with the invention.

CD123 is another receptor associated with myeloid blasts and LSCs but with low level expression on other cell types, such as healthy hematopoietic stem cells and lymphocytes. AML cells with high CD123 expression exhibit higher proliferation and higher expression of CD123 among AML blasts is associated with lower complete remission rates and poorer overall survival. Agents targeting CD123 therefore represent another suitable combination for use with treatment according to the present invention. Anti-CD123 agents suitable for use in treating AML include toxin-linked natural ligand (e.g. DT₃₈₈IL3) and antibodies such as CSL360, CSL362 and MGD006 (a modified antibody (DART)). Therefore, in certain embodiments of the invention, the patient is administered an anti-CD123 agent, for example an anti-CD123 antibody, as part of a combination therapy with the anti-CD70 antibody (and optionally NMI) in accordance with the invention.

Treatment of AML with an anti-CD70 antibody (as a monotherapy or together with an NMI (e.g. aza)) can be further advantageously combined in a combination therapy with other AML therapeutic agents, for example E-selectin inhibitors, FMS-like tyrosine kinase receptor 3 (FLT3) inhibitors, cyclin-dependent kinase inhibitors, BCL-2 inhibitors, aminopeptidase inhibitors and JAK/STAT inhibitors.

As already described, other AML therapeutic agents include cytarabine, anthracycline compounds (e.g. daunorubicin, idarubicin), and hydroxyurea.

### Anti-CD70 antibody or antigen binding fragment thereof

In all aspects of the invention described herein, the subject to be treated is administered an anti-CD70 antibody or antigen binding fragment thereof. As used herein, an "antibody" refers to immunoglobulins having a combination of two heavy and two light chains which have specific immunoreactive activity to an antigen of interest (e.g. human CD70). The terms "CD70 antibodies" or "anti-CD70 antibodies" are used interchangeably herein to refer to antibodies which exhibit immunological specificity for human CD70 protein. "Specificity" for human CD70 in this context does not exclude cross-reaction with species homologues of CD70.

"Antibody" as used herein encompasses antibodies of any human class (e.g. IgG, IgM, IgA, IgD, IgE) as well as subclasses/isotypes thereof (e.g. IgG1, IgG2, IgG3, IgG4, !gA1). Antibody as used herein also refers to modified antibodies. Modified antibodies include synthetic forms of antibodies, which are altered such that they are not naturally occurring, e.g., antibodies that comprise at least two heavy chain portions but not two complete heavy chains (such as, domain deleted antibodies or minibodies); multispecific forms of antibodies (e.g., bispecific, trispecific, etc.) altered to bind to two or more different antigens or to different epitopes on a single antigen); heavy chain molecules joined to scFv molecules and the like. In addition, the term "modified antibody" includes multivalent forms of antibodies (e.g., trivalent, tetravalent, etc., antibodies that bind to three or more copies of the same antigen).

Antibodies described herein may possess antibody effector function, for example one or more of antibody dependent cell-mediated cytotoxicity (ADCC), complement dependent cytotoxicity (CDC) and antibody dependent cellular phagocytosis (ADCP).

CD70 antibody suitable for use according to all aspects of the invention include ARGX-110 described in WO2012123586 (incorporated herein by reference) and SGN-70 (WO2006113909, and McEarChern et al. Clin Cancer Res 2008;14(23) p7763, both incorporated herein by reference).

A CD70 antibody suitable for use according to all aspects of the invention may also be an antibody drug conjugate (ADC). ADCs are antibodies attached to active agents, for example auristatins and maytansines or other cytotoxic agents. Certain ADCs maintain antibody blocking and/or effector function (e.g. ADCC, CDC, ADCP) while also delivering the conjugated active agent to cells expressing the target (e.g. CD70). Examples of anti-CD70 ADCs include vorsetuzumab mafodotin (also known as SGN-75, Seattle Genetics), SGN-70A (Seattle Genetics), and MDX-1203/BMS936561 (Britsol-Myers Squibb), each of which may be used in accordance with the invention. Suitable anti-CD70 ADCs are also described in WO2008074004 and WO2004073656, each of which is incorporated herein by reference).

An "antigen binding fragment" refers to a polypeptide fragment of an antibody that maintains binding specificity for CD70 and includes : an antibody light chain variable domain (VL); an antibody heavy chain variable domain; a single chain variable fragment or single chain antibody (scFv); a Fab fragment; a F(ab')₂ fragment; a Fd fragment; a Fv fragment; a one armed (monovalent) antibody; diabodies; triabodies; tetrabodies; or any antigen binding molecule including such antigen binding fragments (e.g. a chimeric antigen receptor), or any antigen binding molecule formed by combination, assembly or conjugation of such antigen binding fragments. Fragments can be obtained, for example, by chemical or enzymatic treatment of an intact or complete antibody or antibody chain, or by recombinant means.

As set out in the Examples, effective treatment of AML patients with the CD70 antibody ARGX-110 has been demonstrated. ARGX-110 is an IgG1 anti-CD70 antibody that has been shown to inhibit the interaction of CD70 with its receptor CD27 (Silence et al. MAbs. 2014 Mar-Apr;6(2):523-32, incorporated herein by reference). In particular, ARGX-110 has been shown to inhibit CD70-induced CD27 signalling. Levels of CD27 signalling may be determined by, for example, measurement of serum soluble CD27 as described in Riether et al (J. Exp. Med. 2017 Feb;214(2):359-380) or of IL-8 expression as described in Silence et al (MAbs. 2014 Mar-Apr;6(2):523-32). Without being bound by theory, inhibiting CD27 signalling is thought to reduce activation and/or proliferation of Treg cells, thereby reducing inhibition of anti-tumour effector T cells.

Therefore, in all aspects of the invention, the anti-CD70 antibody may be an antibody that inhibits the interaction of CD70 with its receptor CD27. In certain such embodiments, the anti-CD70 antibody may compete with CD27 for CD70 binding. In certain embodiments, the anti-CD70 antibody may inhibit CD70-induced CD27 signalling. In certain embodiments the anti-CD70 antibody may inhibit Treg activation and/or proliferation.

AGRX-110 has also been demonstrated to deplete CD70-expressing tumour cells. In particular, ARGX-110 has been shown to lyse CD70-expressing tumour cells via antibody dependent cell-mediated cytotoxicity (ADCC) and complement dependent cytotoxicity (CDC), and also to increase antibody dependent cellular phagocytosis (ADCP) of CD70-expressing cells (Silence et al, MAbs. 2014 Mar-Apr;6(2):523-32).

Therefore, in certain embodiments, the anti-CD70 antibody may be an antibody that depletes CD70-expressing cells. In certain embodiments, the anti-CD70 antibody may induce lysis of CD70-expressing cells. In certain embodiments, the anti-CD70 antibody may possess ADCC and/or CDC functionality. In certain embodiments, the anti-CD70 antibody may induce ADCP.

The Fc region of ARGX-110 is a defucosylated Fc region according to the Potelligent^{™} system and ARGX-110 has been described as exhibiting increased ADCC functionality compared to a fucosylated counterpart (Silence et al. MAbs. 2014 Mar-Apr;6(2):523-32). Therefore, in certain embodiments the anti-CD70 antibody is fully or partially defucosylated.

In certain embodiments, the CD70 antibody comprises an Fc domain, CH3 domain or Fc-hinge derived from human IgG1 comprising Lys433, Phe434 and Tyr436 (according to EU numbering). In certain embodiments the anti-CD70 antibody further comprises one or more of Tyr252, Thr254 and Glu256 (according to EU numbering). These residues at these positions have been demonstrated to prolong antibody circulation time.

In certain embodiments, the anti-CD70 antibody is an IgG antibody, preferably an IgG1 antibody.

As already described and in light of the data first provided herein, anti-CD70 antibodies other than ARGX-110 are expected to be effective treatments in accordance with the invention, for example CD70 antibodies that inhibit interaction of CD70 with CD27, compete with CD27 for CD70 binding, inhibit CD70-induced CD27 signalling, inhibit Treg activation and/or proliferation, deplete CD70-expressing cells, induce lysis of CD70-expressing cells, possesses ADCC, CDC functionality, and/or induces ADCP. Alternative CD70 antibodies expected to be useful in treatment according to the invention are known in the art, for example SGN-70 and those described in WO2006044643 and WO2007038637, each incorporated herein by reference. Modified CD70 antibodies such as the ADCs SGN-75, SGN-70A and MDX-1203/BMS936561 are further examples of anti-CD70 antibodies expected to effective in treatments according to the invention.

In certain embodiments non-limiting embodiments the anti-CD70 antibody may comprise the CDR sequences of ARGX-110. That is, in certain embodiments the anti-CD70 antibody comprises a variable heavy chain domain (VH) and a variable light chain domain (VL), wherein the VH and VL domains comprise the CDRs (by Kabat definition):
HCDR3 comprising or consisting of SEQ ID NO:3 (DAGYSNHVPIFDS)
HCDR2 comprising or consisting of SEQ ID NO:2 (DINNEGGTTYYADSVKG)
HCDR1 comprising or consisting of SEQ ID NO:1 (VYYMN)
LCDR3 comprising or consisting of SEQ ID NO:7 (ALFISNPSVE)
LCDR2 comprising or consisting of SEQ ID NO:6 (NTNTRHS), and
LCDR1 comprising or consisting of SEQ ID NO:5 (GLKSGSVTSDNFPT).

In certain embodiments, the VH and/or VL domains of the anti-CD70 antibody exhibit at least 70%, at least 80%, at least 90%, at least 95%, at least 98%, at least 99% sequence identity to the VH and/or VL domains of ARGX-110, respectively (VH: SEQ ID NO: 4; VL: SEQ ID NO: 8). For embodiments wherein the domains of the antibodies or antigen binding fragments are defined by a particular percentage sequence identity to a reference sequence, the VH and/or VL domains may retain identical CDR sequences to those present in the reference sequence such that the variation is present only within the framework regions. In certain embodiments, the anti-CD70 antibody is ARGX-110.

**Table 2**

| ARGX-110 | Sequence | SEQ ID NO |
|---|---|---|
| HCDR1 | VYYMN | 1 |
| HCDR2 | DINNEGGTTYYADSVKG | 2 |
| HCDR3 | DAGYSNHVPIFDS | 3 |
| VH | EVQLVESGGGLVQPGGSLRLSCAASGFTFSVYYMNWVRQAPGKGLEWVSDI NNEGGTTYYADSVKGRFTISRDNSKNSLYLQMNSLRAEDTAVYYCARDAGYS NHVPIFDSWGQGTLVTVSS | 4 |
| LCDR1 | GLKSGSVTSDNFPT | 5 |
| LCDR2 | NTNTRHS | 6 |
| LCDR3 | ALFISNPSVE | 7 |
| VL | QAVVTQEPSLTVSPGGTVTLTCGLKSGSVTSDNFPTWYQQTPGQAPRLLIYNT NTRHSGVPDRFSGSILGNKAALTITGAQADDEAEYFCALFISNPSVEFGGGTQL TVLG | 8 |

### EXAMPLES

### Example 1: Effect of anti-CD70 antibody monotherapy, or in combination with decitabine, on human AML LSCs grafted into mice

NSG mice were transplanted with 5x10⁶ CD45^{dim}SSC^{lo} human AML cells. 32 days after engraftment (engraftment in PB: 14.45 +/- 0.95%), NSG mice were randomized to treatment with vehicle (Veh), aCD70 mAb (aCD70, ARGX-110, 10 mg/kg), decitabine (D, 1,5 mg/kg/d) or the combination (aCD70/D) for 5 days and bone marrow, spleen and blood were analyzed.

Both anti-CD70 and decitabine alone resulted in reduced total engraftment in the bone marrow, spleen and blood (Figure 1). The combination of anti-CD70 and decitabine resulted in enhanced reduction of the percentage of engrafted human cells compared to either therapy alone (Figure 1).

The combination therapy also reduced CD34+ AML cells (a marker of progenitor cells) in the bone marrow better than either decitabine or anti-CD70 alone (Figure 1). In addition, anti-CD70 treatment reduced both the number of CD34+CD38- cells and of CD34+CD45- cells, both cell populations thought to be markers for leukemic stem cells (LSCs). Whilst decitabine alone showed minimal effect at reducing LSC numbers, the combination of anti-CD70 and decitabine resulted in a greatly enhanced reduction of LSCs (Figure 1).

### Example 2: Hypomethylating agents (HMAs) uprequlate CD70 expression on primary AML stem cells ex vivo and in vivo, correlating with enhanced reduction in AML colony formation by anti-CD70 antibody combined with an HMA.

The finding that anti-CD70 antibody treatment in combination with a nucleoside metabolic inhibitor (NMI), for example hypomethylating agent decitabine, results in an enhanced reduction in AML blast engraftment in mice, was further investigated in primary human AML LSCs.

The effect of NMI treatment (e.g. HMAs such as azacitidine or decitabine) on CD70 expression by AML LSCs was investigated. CD34+ CD38- cells were isolated from AML patients and cultured in the presence of 0.5mM decitabine or vehicle.

The data in Figure 2A demonstrate that CD70 expression by AML LSC cells is increased when the cells are cultured with decitabine. This increase in CD70 expression in response to decitabine occurs in cells taken from AML patients across all disease risk categories (favourable, intermediate and adverse) and when measured at the protein or transcriptional level (Figure 2C and 2D).

Significantly, the increase in CD70 expression in response to hypomethylating agents (HMA) was observed *in vivo.* LSCs taken from patients newly diagnosed with AML and treated with decitabine or azacitidine showed an increase in CD70 expression in response to HMA treatment when compared to the expression level at diagnosis (Figure 2E and 2F).

In light of the increased expression of CD70 in response to HMAs, the effect of the anti-CD70 antibody/decitabine combination on AML LSCs was investigated *ex vivo.*

Figure 3A shows that both anti-CD70 and decitabine monotherapy reduce the number of colonies formed by AML LSCs in a colony plating assay. Notably, the combination of anti-CD70 and decitabine reduced colony formation more than either monotherapy alone. This effect was observed across all disease risk categories (favourable (P6), intermediate (P8) and adverse (p11)). The re-plating capacity of the AML LSCs was also reduced (Figure 3C).

Significantly, the anti-CD70 antibody did not adversely affect healthy stem cells. Figure 3D-F show anti-CD70 as a monotherapy had no effect on healthy cells compared to vehicle alone, and when used in combination had no effect compared to decitabine used alone.

These data support use of anti-CD70 as a monotherapy for AML, as well as in combination with a nucleoside metabolic inhibitor (NMI), for example an HMA such as azacitidine or decitabine.

### Example 3: Phase I/II trial of anti-CD70 antibody ARGX-110 in combination with standard doses of AZA in subjects with previously untreated AML and high risk MDS

A phase I/II clinical trial was begun to investigate the efficacy/clinical benefits and safety and tolerability of ARGX-110 in combination with standard doses of AZA in subjects with previously untreated AML and high risk MDS who are eligible for AZA treatment.

### Trial regimen protocol and sample assays

The study included a screening phase (between Day -35 and Day -14), a loading dose of ARGX-110 (Day -14) and an open-label treatment phase during which subjects visited the study centre for administration of the study drug (Day -14 until disease progression), and end-of-treatment (EOT) evaluations performed within 7 days after the last ARGX-110 treatment. Additional follow-up evaluations were scheduled at 30 and 60 days (± 7 days) after the EOT date. The 60-days follow-up visit was also the end-of-study (EOS) visit.

Male and female subjects, at least 18 years of age, with newly diagnosed histologically confirmed (bone marrow biopsy) AML or high-risk myelodysplastic syndrome (MDS) with blast-count >20%, who were not fit for standard intensive chemotherapy were eligible for enrolment in the study. Subjects were required to have a life expectancy of 3 or more months, and Eastern Cooperative Oncology Group (ECOG) performance status of 0 - 2 at screening. Subjects received a loading dose of ARGX-110 on Day -14, followed by administration of ARGX-110 in combination with standard doses of AZA. Doses of ARGX-110 were administered intravenously every 2 weeks (loading dose at Day -14 and further doses on Days 3 and 17) in combination with standard doses of AZA to determine the dose limiting toxicity (see Figure 4). The first patient of each cohort was monitored until day 7 before a second patient was included. Since no significant adverse events occurred within this timeframe the other patients of each cohort were enrolled. The ARGX-110 dose level in the first cohort in Phase I was 1 mg/kg body weight.

All subjects in the study received a single loading dose of ARGX-110 on Day -14, at the same dose level as the bi-weekly dose. Subjects in Phase I of the study received one of the following treatments:
- Cohort 1: 1 mg/kg body weight IV on Days 3 and 17 of a 28-day cycle
- Cohort 2: 3 mg/kg body weight IV on Days 3 and 17 of a 28-day cycle
- Cohort 3: 10 mg/kg body weight IV on Days 3 and 17 of a 28-day cycle
- Cohort 4: 20 mg/kg body weight IV on Days 3 and 17 of a 28-day cycle

The infusion was started at a rate of 10 mL/hr. The rate was then increased according to the tolerability of the drug by the patient.

Clinical response to treatment was categorised according Table 3:

**Table 3**

| **Response Criteria** | **Definition** |
|---|---|
| Complete remission (CR)* | Bone marrow blasts < 5%; absence of blasts with Auer rods; absence of extramedullary disease; absolute neutrophil count > 1.0 x 10⁹/L (1000/µL); platelet count > 100 x 10⁹/L (100.000/µL); independence of red cell transfusions |
| CR with incomplete recovery (CRi) | All CR criteria except for residual neutropenia (< 1.0 × 10⁹/L [1000/µL]) or thrombocytopenia (< 100 × 10⁹/L [100.000/µL]) |
| Morphologic leukemia-free state (MLFS) | Bone marrow blasts < 5%; absence of blasts with Auer rods; absence of extramedullary disease; no hematologic recovery required |
| Partial remission (PR) | Relevant in the setting of phase I and II clinical trials only; all hematologic criteria of CR; decrease of bone marrow blast percentage to 5% to 25%; and decrease of pretreatment bone marrow blast percentage by at least 50% |

| Treatment failure | |
|---|---|
| Resistant disease (RD) | Failure to achieve CR or CRi (general practice; phase II/III trials), or failure to achieve CR, CRi, or PR (phase I trials); only includes subjects surviving > 7 days following completion of initial treatment, with evidence of persistent leukemia by blood and/or bone marrow examination |
| Death in aplasia | Deaths occurring > 7 days following completion of initial treatment while cytopenic; with an aplastic or hypoplastic bone marrow obtained within 7 days of death, without evidence of persistent leukemia |
| Death from indeterminate cause | Deaths occurring before completion of therapy, or < 7 days following its completion; or deaths occurring > 7 days following completion |
| Relapse | Bone marrow blasts > 5%; or reappearance of blasts in the blood; or development of extramedullary disease |

| | |
|---|---|
| *All criteria need to be fulfilled; marrow evaluation should be based on a count of 200 nucleated cells in an aspirate with spicules; if ambiguous, consider repeat exam after 5 to 7 days; flow cytometric evaluation may help to distinguish between persistent leukaemia and regenerating normal marrow; a marrow biopsy should be performed in cases of dry tap, or if no spicules are obtained; no minimum duration of response required. | |

The serum concentrations of ARGX-11 0 were analysed using a validated enzyme-linked immunosorbent assay (ELISA) method. The following pharmacokinetic parameters were assessed: • Cₘₐₓ: maximum observed concentration; C_{trough}: trough concentration; AUC∞: area under the serum concentration-time curve from time zero to infinity; AUCₜₐᵤ: area under the serum concentration-time curve during the dosing interval; V_{d}: apparent volume of distribution; CL: total systemic clearance of drug after IV administration; t1/2: half-life.

For all subjects in the study, venous blood samples were drawn to assess anti-drug antibodies (ADAs). The immunogenicity of ARGX-110 was evaluated in serum samples using an ELISA method that can detect any class of ADA. Reactive samples were analysed in a confirmatory assay for verification of specificity.

Evaluation of biomarkers may be performed on bone marrow aspirates and/or whole blood collected at time points as specified in Figure 5. Pharmacodynamics was examined by measuring a series of biomarkers including:
Molecular genetics: characterization of CD70 and CD11a promoter methylation, to identify recurring AML genomic aberrations, and for genomic DNA analyses of treatment effect on disease and target pathology.
Gene expression: characterization of mRNA levels of CD70, disease and drug effect markers.
Flow cytometry (FACS): additional characterization of CD70 (e.g. pre-ARGX-110 treatment, post-relapse) and CD27 expression, and drug effect (e.g. blast, NK and T-cells), and minimum residual disease (MRD) analysis.
Serum protein quantification: additional characterization of sCD27, disease and drug effect markers (e.g. IL-8). If infusion-related reactions are more severe than in earlier ARGX-110 trials, inflammatory cytokine analyses can be assessed.

Stemness determinations were performed on mononuclear cells purified from blood or bone marrow. Depending on the number of cells that were harvested, readouts included Numb staining (to determine the ratio of asymmetric/symmetric division), cell and *in vivo* studies (evaluate stem cell potential by e.g. CFU methylcellulose colony assays or survival studies of NSG mice injected with patient mononuclear cells) or gene expression analysis.

Minimal residual disease (MRD) assessments were performed on bone marrow aspirates and/or whole blood collected at time points as specified in Figure 5. Flow cytometry was utilized as the primary method of MRD analysis. Suitable flow cytometry markers for MRD assessment and determining AML subtype include CD16, CD13, CD34, CD117, CD11b, CD10, HLA-DR, CD45, CD35, CD64, IREM-2, CD36, CD105, CD14, CD33, CD71, CD36, CD105, CD33, CD71, cTdT, CD56, CD7, CD19, cMPO, cLactoferrin, cLysozyme. An additional panel was used as a part of the trial, focusing on CD70, CD27 expression as well as markers indicative of stem cell potential or myeloid differentiation: CD27, CD70, CD34, CD117, CD11b, HLA-DR, CD45, CD38, and CD123. When available, flow cytometry results were compared to other molecular approaches such as polymerase chain reaction (PCR) test.

Bone marrow aspirates or whole blood may be utilized for immunophenotyping (performed by flow cytometry or mass cytometry) which includes analyses of NK cells, and T cells as well as other potential immune cell subpopulations.

### Results

### Overall clinical results

Significantly, more than 90% of patients recruited to the trial responded to anti-CD70 therapy (10/11 patients who have been treated for sufficient time to asses response).

Table 4 shows the best response of those patients for whom sufficient time has been spent on the trial to assess response. Across each of the 1 mg/kg, 3 mg/kg and 10 mg/kg cohorts, 2 of the 3 patients in each cohort showed complete remission. In Cohort 1 the third patient achieved complete remission with incomplete haematological recovery.

A response level of greater than 90% is in stark contrast to the response rate of approximately 25% seen with aza alone (Dombret et al. Blood 2015 (Blood. 2015;126(3):291-299, incorporated herein by reference).

**Table 4**

| | **Risk** | **Best response to date** | **Time to best response** |
|---|---|---|---|
| **Cohort 1 (1mg/kg)** | Adverse | CRi | C5D1 |
| | Intermediate | CR | EOT |
| | Adverse | CR | C7D1 |
| **Cohort 2 (3mg/kg)** | Intermediate | CR | C4D1 |
| | Intermediate | CR | C7D2 |
| | Adverse | PR | EOT |
| **Cohort 3 (10mg/kg)** | Intermediate | CR | C3D23 |
| | Intermediate | CR | C4D1 |
| | Adverse | MLFS | C1D1 |
| **Cohort 4 (20mg/kg)** | Adverse | CRi | C3D1 |
| | Adverse | PR | C1D1 |
| | Intermediate | No response to date | |

Notably, morphologic leukemia-free state (MLFS) was reached after monotherapy with ARGX-1 10 alone. A 71 year old patient in Cohort 2 with 20% bone marrow blasts at recruitment achieved and a 74 year old patient in Cohort 3 with >50% bone marrow blasts at recruitment achieved MLFS characterised by no blasts by time-point C1D1 - i.e. after the loading dose with ARGX-110 and prior to the first aza dose.

This clearly demonstrates that anti-CD70 antibody therapy alone can be an effective treatment for AML.

Furthermore, complete remission was also induced by combination ARGX-110/aza therapy. Six patients across each of Cohorts 1-4 achieved complete remission following combination therapy, with a further two patients achieving CRi following combination therapy.

Of further importance is the fact that combination treatment allowed one patient to progress to bone marrow transplant. This is significant due to the fact that many AML patients, particularly the elderly patients, are typically unable to undergo transplant due to the aggressive nature of the conventional therapies required to meet the transplant criteria. The fact that the combination treatment was sufficiently effective to allow a 75 year old AML patient to progress to transplant shows the advantages of the combination therapy over conventional treatment.

### Mono and combination therapy reduce blasts in bone marrow and peripheral blood

For each patient, the number of blasts in bone marrow and peripheral blood were assessed at various time-points as outlined in Figure 5.

To assess bone marrow blasts, bone marrow aspirates taken at the various time points were analysed. Methylcellulose colony assays using FACS sorted monocytes (mononuclear cells (CD45^{dim}) were selectively sorted by excluding doublets, dead cells and lymphocytes using Annexin V and antibodies for CD19 and CD4/CD8) were used to assess the number of LSCs in the bone marrow. In addition, bone marrow blast percentages were also assessed by cell morphology and flow cytometric analysis.

The bone marrow blast results for each patient are shown collated in Figure 6. These data show that for the majority of patients, bone marrow blast percentage was at least partially or completely reduced by C1D1 - i.e. following ARGX-110 monotherapy.

Furthermore, once aza therapy is begun (from C1D1) the bone marrow blast percentage is further reduced and maintained at a low level for the remainder of the treatment. This is the case regardless of whether cytomorphological or flow based blast assays are used.

It is particularly notable that bone marrow blasts as measured according to minimum residual disease (MRD) criteria are shown to be significantly reduced by both ARGX-110 monotherapy and ARGX-110 and aza combination therapy, given the clinical importance of MRD assessment. Two patients (one in Cohort 1 and one in Cohort 2) reached MRD-status.

Similar results are also observed when peripheral blood blast percentages are assessed (Figure 7). Changes in a patient's peripheral blood data are more susceptible to exterior factors, thus leading to more variation in the data. Nevertheless, the peripheral blood (PB) results correspond to the changes observed in the bone marrow following mono and combination therapy (Figure 7).

Figure 7 shows that, similarly to the data from bone marrow, PB blast percentage in general is reduced following ARGX-110 monotherapy (i.e. at C1D1 compared to earlier data points) and then further reduced under the combination therapy (from C1D1 onwards). Again, this effect is observed regardless of whether cytomorphological or flow based blast assays are used, and is evident when using MRD assessment criteria.

Anti-CD70 antibody monotherapy and the combination of anti-CD70 plus an HMA such as aza are thus each able to reduce blast percentage in the bone marrow and peripheral blood and, in the case of the combination therapy, maintain this reduced percentage over a number of time points.

### Mono and combination therapy increase myeloid differentiation of LSCs

The persistence of leukaemic stem cells (LSCs) in AML represents a significant hurdle for the successful treatment of AML patients. LSCs are responsible for disease relapse in AML, and their number is maintained through a process of symmetric cell division - that is, each LSC divides symmetrically to generate two daughter LSCs. Such symmetric division is a driving factor in AML aggressiveness and patient relapse.

An alternative cell fate for LSCs is to undergo myeloid differentiation. In this process, an LSC undergoes *asymmetric* division, generating a myeloid differentiated daughter cell alongside a daughter stem cell.

Such asymmetric division greatly reduces the pool of LSCs in the patient and increased asymmetric division is indicative of improved response by the patient.

The level of asymmetric division in a population of LSCs can be determined by measuring the level of a cell fate determinant such as the Numb protein (Riether et al. J Exp Med. 2017 Feb; 214(2): 359-380, incorporated herein by reference). Increased Numb expression is indicative of an increase in asymmetric division and therefore an increase in myeloid differentiation and decreased LSC populations.

Figure 8 shows that Numb expression is increased following ARGX-110 monotherapy (C1D1) and then further increased under the combination ARGX-110/aza therapy, indicating both therapies increase asymmetric division and thus myeloid differentiation of LSCs.

These data indicate that not only does the anti-CD70 monotherapy and aza combination therapy reduce patient blast count by modulating the CD70-CD27 pathway (thereby promoting T cell responsiveness), but that the anti-CD70 antibody is also promoting LSC differentiation. This differentiation reduces the stem cell population and thus further contributes to the reduction in blasts observed in the trial patients.

### Monotherapy and combination therapy both decrease ex vivo colony formation

To further explore the impact of the mono and combination therapies on the LSC populations in patients, methylcellulose colony assays using FACS sorted *ex vivo* monocytes were performed. Mononuclear cells (CD45^{dim}) were selectively sorted by excluding doublets, dead cells and lymphocytes using Annexin V and antibodies for CD19 and CD4/CD8 and serially plated on methylcellulose for 14 days.

Figure 9A-E provides representative data for colony formation per well (and thus the number of LSCs) before treatment (SCR) and after monotherapy with ARGX-110 (Time-point 0, corresponding to C1D1)

Figure 9F provides representative data for colony formation per well (and thus the number of LSCs) before treatment, after monotherapy with ARGX-110 (i.e. at C1D1) and after the combination therapy (C4D1). These data correspond to approximately a 24-fold reduction in LSC frequency compared to baseline levels.

In addition, not only does therapy reduce the number of LSCs, but the number of cells per colony is also reduced, indicating that the proliferative potential of the LSCs is also reduced following therapy (data not shown).

Again, these data demonstrate that circulating LSC levels are reduced following monotherapy with ARGX-110 and then reduced further following the combination of ARGX-110 and aza. Moreover, the proliferative potential of these LSCs is also reduced by therapy.

### Monotherapy and combination therapy both decrease soluble CD27 levels

Soluble CD27 (sCD27) can serve as a biomarker for the extent of CD70/CD27 interactions.

CD70/CD27-mediated signalling is thought to promote aberrant cell divisions and high levels of serum sCD27 are correlated with poor prognosis of AML patients. In addition, sCD27 is thought to correlate with the percentage of blast cells in bone marrow, as well as to serve as a marker for the sternness of a patient's blasts, with increased sCD27 indicating increased levels of stemness (Riether et al. J. Exp. Med. 2017 Feb;214(2):359-380).

To assess the effect of anti-CD70 monotherapy and combination therapy on the CD27/CD70 interaction as well as to further explore the effect on blast percentage and stemness, patients' soluble CD27 levels were also measured.

Figure 10 shows representative sCD27 levels for a patient during the treatment cycle. Monotherapy with ARGX-110 (C1D1) reduces sCD27 levels compared to before treatment ("Loading"), and this reduction continues to decrease during combination therapy such that it reaches levels representative of a healthy patient.

These data demonstrate that both mono and combination therapy are inhibiting the CD27/CD70 interaction and reducing the blast percentages and stemness, in accordance with the results from other assays.

In addition, Figure 10 shows that sCD27 levels rise after the end of treatment (EOT). This is likely driven by an increase in the CD27/CD70 interaction once the anti-CD70 antibody is removed, and indicates that sCD27 can be used as an effective marker for anti-CD70 drug engagement in a patient.

### ARGX-110 therapy does not increase toxicity

A significant factor in the suitability of a therapeutic regimen is the associated toxicity to the patient. Advantageously, the anti-CD70 antibody ARGX-110 did not result in any observed increase in toxicity, either as a monotherapy or when used in combination with aza.

For each of the 1 mg/kg, 3 mg/kg, and 10 mg/kg cohorts, adverse events and haematological toxicity was not different to that reflecting the usual azacitidine safety profile. This is particularly surprising and advantageous, since addition of a combination therapeutic agent frequently results in an increase in toxicity. However, with the ARGX-110/aza combination, no increase in toxicity was observed and a significant increase in efficacy induced.

**Table 5**

| **Grade 3-5 adverse events In Phase 1 patients** | **Cohort 1 (1mg/kg)** | **Cohort 2 (3mg/kg)** | **Cohort 3 (10mg/kg)** | **Cohort 4 (20 mg/kg)** | **Total** |
|---|---|---|---|---|---|
| | **Events (patients)** | **Events (patients)** | **Events (patients)** | **Events (patients)** | |
| Anaemia | 4(1) | 10(3) | | 2(1) | 16 |
| Thrombocytopenia | 8 (2) | 3 (3) | 2(1) | 2(2) | 15 |
| Neutropenia | 2(1) | 4(1) | | | 6 |
| Febrile Neutropenia | 2(2) | | 1 (1) | 1(1) | 4 |
| Hypertension | | 2(1) | | | 2 |
| Proctitis | | 2(1) | | | 2 |
| Leukopenia | 1 (1) | 1 (1) | | | 1 |
| Lung infection | 1 (1) | | | | 1 |
| Pleuro-pericarditis | 1 (1) | | | | 1 |
| Constipation | | 1 (1) | | | 1 |
| Fever | | 1 (1) | | | 1 |
| Hypokalaemia | | 1 (1) | | | 1 |
| Tooth Infection | | 1 (1) | | | 1 |
| Disease Progression | | | 1(1) | | 1 |

### Pharmacokinetic (PK) data

The serum concentration of ARGX-110 was analysed using a validated enzyme-linked immunosorbent assay (ELISA) method. Figure 11 provides individual patient PK plots for the 10mg/kg cohort. The PK plot presents data for ARGX-110 cycle 1 (D-14 pre-dose until Cycle1 D1 pre-dose).

Overall cohorts, the pharmacokinetics of ARGX-110 showed dose proportionality of Cmax and AUC over the dose range studied and a half-life of approximately 9.2 days at 10 mg/kg.

Concentration of ARGX-110 in bone marrow aspirates was measured in limited samples and compared to the matching plasma samples. The ARGX-110 concentration in bone marrow aspirates was comparable to the plasma levels for patient 1001005 and 1001007 in dose cohort 3 mg/kg and 10 mg/kg respectively.

**Table 6**

| **Patient** | **Dose cohort** | **Sample** | **C_{ARGX-110} BM aspirate** | **C_{ARGX-110} plasma** |
|---|---|---|---|---|
| 1001005 | 3 mg/kg | Cycle 7 Day 2 | 33,1 µg/ml | 37,3 µg/ml |
| 1001007 | 10 mg/kg | Cycle 4 Day 1 | 117,7 µg/ml | 126,6 µg/ml |

### Patient characteristics and case studies

Table 7 provides a summary of the newly diagnosed AML patients recruited to each cohort:

**Table 7**

| **Patient Characteristic** | | **1 mg/kg** | **3 mg/kg** | **10 mg/kg** | **20 mg/kg** | **Total** |
|---|---|---|---|---|---|---|
| **Age** | | | | | | |
| | **Mean (range)** | 78 (71-80) | 75 (71-84) | 71 (64-75) | 75 (72-77) | 74 (64-84) |
| **Gender (Male : Female)** | | 2:1 | 1:2 | 2:1 | 2:1 | 7:5 |

| **Risk (ELN 2017)** | | | | | | |
|---|---|---|---|---|---|---|
| | **Intermediate** | 1 | 2 | 2 | 1 | 6 |
| | **Adverse** | 2 | 1 | 1 | 2 | 6 |

| **Bone Marrow Blasts** | | | | | | |
|---|---|---|---|---|---|---|
| | **Median % (range)** | 51.3 (24-90) | 40 (20-60) | 70 (50-80) | 50 (22-80) | 52.7 (20 -90) |

| **AML Classification (WHO 2016)** | | | | | | |
|---|---|---|---|---|---|---|
| | **NOS** | 0 | 1 | 3 | 0 | 4 |
| | **With Myelodysplasia-related changes** | 2 | 2 | 0 | 2 | 6 |
| | **Therapy related myeloid neoplasm** | 1 | 0 | 0 | 0 | 1 |
| | **Recurrent genetic abnormalities** | 0 | 0 | 0 | 1 | 1 |
| **French American British Classification** | | M4, M1, M2 | M4, M5,M2 | M1, M2, M5a | M2, M2, M4 | |

### The following are case studies of individual patients recruited to the trial.

Patient 1001001 is an 80 year old female with therapy-related AML 5 years after adjuvant chemotherapy for breast cancer with blast counts of 90% in bone marrow (BM) and 80% in peripheral blood (PB) (39.5 G/L), FAB subtype: M4 myelomonocytic and WHO 2016 classification: Provisional subentity: AML with mutated RUNX1 . Patient was treated according to protocol in the 1mg/kg therapy arm.

Bone marrow aspirate taken at day 1 was compared to bone marrow aspirate taken before administration of the ARGX-110 loading dose. Methylcellulose colony assays using FACS sorted monocytes (mononuclear cells (CD45^{dim}) were selectively sorted by excluding doublets, dead cells and lymphocytes using Annexin V and antibodies for CD19 and CD4/CD8) were used to assess the number of LSCs in the bone marrow.

The results showed a single dose of ARGX-110 reduced the number of LSCs in the bone marrow by a factor of 140,000 (calculation of stem cell frequency via ELDA: Extreme Limiting Dilution Analysis (http://bioinf.wehi.edu.au ; Hu &Smyth (2009) ELDA: Journal of Immunological Methods 347, 70-78.).

In addition, cell morphology and flow cytometric analysis of the blast cell populations demonstrated a reduction in bone marrow and in peripheral blood following a single dose (loading dose) of ARGX-110. Following continued treatment in combination with AZA according to the schedule of Figure 5, BM blasts had reduced to 2.8% (according to flow cytometry) by C3D1 (i.e. after 2 cycles of treatment). The patient's clinical response assessment at this time point was therefore complete remission with incomplete recovery (CRi). The minimal residual disease analysis (MRD) was determined to be 0.2%. CRi status by bone marrow aspirate and by MRD assessment was maintained until at least C5D1. In peripheral blood, blasts were cleared by following the first complete treatment cycle (C2D1). By C3D1 the MRD for peripheral blood was 0.3%, and by C4D1 peripheral blood blast percentage was 0.8% blasts by flow cytometry and 0% by cell morphology. At C4D17 the blast cell number in peripheral blood was sufficiently low to be classed as MRD-negative.

Patient 1001002 is a 75 year old male with AML with myelodysplasia-related changes (WHO classification; M1/M2 AML according to FAB classification). The patient exhibited 40% bone marrow blasts prior to treatment. Patient was treated according to protocol in the 1mg/kg therapy arm.

Bone marrow aspirate taken at day 1 was compared to bone marrow aspirate taken before administration of the ARGX-110 loading dose and assessed using methylcellulose colony assays on monocytes. Similarly to patient 1001001, a single dose of ARGX-110 monotherapy reduced the number of LSCs in the bone marrow. In patient 1001002, LSCs were reduced by a factor of 2 following the loading dose of ARGX-110. Colony formation was decreased by greater than 50% at all informative serial dilution concentrations and cell number per colony was also reduced.

Following a single dose of ARGX-110, percentage of total blasts in bone marrow appeared to be reduced when assessed by flow cytometry, but an increase was observed when assessed by cell morphology.

Similarly to patient 1001001, overall blast percentage in peripheral blood fell following the ARGX-110 loading dose (C1D1 vs screening) and continued to decrease during cycle 1, approaching zero by the end of cycle 1 (C2D1). By C2D17, the percentage of blasts in peripheral blood was 0.8% by flow cytometry and 0% by cell morphology. After 3 cycles (C3D17) peripheral blood counts had recovered (Hb 10.2 g/dL; platelets 128 G/L; absolute neutrophil count (ANC) 0.97 G/L) and bone marrow analysis at C4D1 indicated CRi.

Patient 1001003 is a 77 year old male with AML with myelodysplasia-related changes by WHO classification (AML-M2 by FAC classification) and 24% bone marrow blasts. By 1 cycle (C2D1) the patient exhibited no peripheral blasts and was recovering after a brief thrombopenia and lymphopenia (platelets 98 G/L; ANC 0.38 G/L; Hb 9.2 g/dL). Blast percentage at C1D1 was stable.

The data from these patients demonstrates that monotherapy treatment with an anti-CD70 antibody (ARGX-110) reduces the number of leukemic stem cells in the bone marrow of AML patients, and can reduce the total blast percentage in the bone marrow and in the peripheral blood. Combined therapy with an anti-CD70 antibody (ARGX-110) and a nucleoside metabolic inhibitor (azacitidine) further reduces the total blast percentage in the bone marrow and in the peripheral blood to the extent that the patient can be categorised as free of minimum residual disease, an outcome not typically observed with azacitidine therapy alone.

## Claims

1. An anti-CD70 antibody or antigen-binding fragment thereof for use in a method of treating a CD70-expressing tumor or hematological malignancy in a subject, the method comprising:
administering to the subject one or more doses of the anti-CD70 antibody or antigen-binding fragment thereof, and
administering to the subject a nucleoside metabolic inhibitor (NMI) selected from azacitidine and decitabine,
wherein the anti-CD70 antibody comprises a variable heavy chain domain (VH) and a variable light chain domain (VL), wherein the VH and VL domains comprise the CDRs:
HCDR3 comprising or consisting of SEQ ID NO:3 (DAGYSNHVPIFDS),
HCDR2 comprising or consisting of SEQ ID NO:2 (DINNEGGTTYY ADSVKG),
HCDR1 comprising or consisting of SEQ ID NO:1 (VYYMN),
LCDR3 comprising or consisting of SEQ ID NO:7 (ALFISNPSVE),
LCDR2 comprising or consisting of SEQ ID NO:6 (NTNTRHS), and
LCDR1 comprising or consisting of SEQ ID NO:5 (GLKSGSVTSDNFPT).

2. A nucleoside metabolic inhibitor (NMI) selected from azacitidine and decitabine for use in a method of treating a CD70-expressing tumor or hematological malignancy in a subject, the method comprising:
administering to the subject the nucleoside metabolic inhibitor (NMI), and
administering to the subject one or more doses of an anti-CD70 antibody or antigen-binding fragment thereof,
wherein the anti-CD70 antibody comprises a variable heavy chain domain (VH) and a variable light chain domain (VL), wherein the VH and VL domains comprise the CDRs:
HCDR3 comprising or consisting of SEQ ID NO:3 (DAGYSNHVPIFDS),
HCDR2 comprising or consisting of SEQ ID NO:2 (DINNEGGTTYY ADSVKG),
HCDR1 comprising or consisting of SEQ ID NO:1 (VYYMN),
LCDR3 comprising or consisting of SEQ ID NO:7 (ALFISNPSVE),
LCDR2 comprising or consisting of SEQ ID NO:6 (NTNTRHS), and
LCDR1 comprising or consisting of SEQ ID NO:5 (GLKSGSVTSDNFPT).

3. A combination comprising an anti-CD70 antibody or antigen binding fragment thereof and a nucleoside metabolic inhibitor (NMI) selected from azacitidine and decitabine for use in a method of treating a CD70-expressing tumor or hematological malignancy in a subject, the method comprising:
administering to the subject one or more doses of the anti-CD70 antibody or antigen-binding fragment thereof, and
administering to the subject the nucleoside metabolic inhibitor (NMI),
wherein the anti-CD70 antibody comprises a variable heavy chain domain (VH) and a variable light chain domain (VL), wherein the VH and VL domains comprise the CDRs:
HCDR3 comprising or consisting of SEQ ID NO:3 (DAGYSNHVPIFDS),
HCDR2 comprising or consisting of SEQ ID NO:2 (DINNEGGTTYY ADSVKG),
HCDR1 comprising or consisting of SEQ ID NO:1 (VYYMN),
LCDR3 comprising or consisting of SEQ ID NO:7 (ALFISNPSVE),
LCDR2 comprising or consisting of SEQ ID NO:6 (NTNTRHS), and
LCDR1 comprising or consisting of SEQ ID NO:5 (GLKSGSVTSDNFPT).

4. The anti-CD70 antibody or antigen-binding fragment thereof for use according to claim 1, the NMI for use according to claim 2, or the combination for use according to claim 3, wherein the method reduces the percentage of blasts in the bone marrow and/or peripheral blood of the subject.

5. The anti-CD70 antibody or antigen-binding fragment thereof for use according to claim 1 or claim 4, the NMI for use according to claim 2 or claim 4, or the combination for use according to claim 3 or 4, wherein the anti-CD70 antibody or antigen-binding fragment thereof is in a pharmaceutical composition comprising a pharmaceutically acceptable excipient or carrier.

6. The anti-CD70 antibody or antigen-binding fragment thereof for use according to any one of claims 1, 4 and 5, the NMI for use according to any one of claims 2, 4, and 5, or the combination for use according to any one of claims 3-5, wherein the subject is not eligible for standard intensive chemotherapy prior to treatment.

7. The anti-CD70 antibody or antigen-binding fragment thereof for use according to any one of claims 1 and 4-6, the NMI for use according to any one of claims 2 and 4-6, or the combination for use according to any one of claims 3-6, wherein the method further comprises the step of conducting a hematopoietic stem cell transplant on the subject.

8. The anti-CD70 antibody or antigen-binding fragment thereof for use according to any one of claims 1 and 4-7, the NMI for use according to any one of claims 2 and 4-7, or the combination for use according to any one of claims 3-7, wherein the subject is 60 years old or older, optionally 75 years old or older.

9. The anti-CD70 antibody or antigen-binding fragment thereof for use according to any one of claims 1 and 4-8, the NMI for use according to any one of claims 2 and 4-8, or the combination for use according to any one of claims 3-8, wherein the anti-CD70 antibody or antigen-binding fragment comprises a VH domain at least 80% identical to SEQ ID NO:4 and/or a VL domain at least 80% identical to SEQ ID NO:S.

10. The anti-CD70 antibody or antigen-binding fragment thereof for use according to any one of claims 1 and 4-9, the NMI for use according to any one of claims 2 and 4-9, or the combination for use according to any one of claims 3-9, wherein the anti-CD70 antibody or antigen-binding fragment comprises a VH domain comprising or consisting of SEQ ID NO:4 and/or a VL domain comprising or consisting of SEQ ID NO:8.

11. The anti-CD70 antibody for use according to any one of claims 1 and 4-10, the NMI for use according to any one of claims 2 and 4-10, or the combination for use according to any one of claims 3-10, wherein the anti-CD70 antibody is an lgG1 antibody.

12. The anti-CD70 antibody, NMI, or combination for use according to claim 11, wherein the anti-CD70 antibody comprises a VH domain consisting of SEQ ID NO:4 and a VL domain consisting of SEQ ID NO:8.

13. The anti-CD70 antibody, NMI, or combination for use according to claim 12, wherein the anti-CD70 antibody is administered at a dose in a range of from 0.1 mg/kg to 25 mg/kg per dose.

14. The anti-CD70 antibody, NMI, or combination for use according to claim 13, wherein the administration of each said dose of the anti-CD70 antibody is separated by 10-20 days, optionally 12-18 days, optionally 14-17 days.

15. The anti-CD70 antibody or antigen-binding fragment thereof, NMI, or combination for use according to any one of the preceding claims, wherein the method treats a hematological malignancy in the subject selected from acute myeloid leukemia (AML) and myelodysplastic syndrome (MDS).
